# EUROPEAN PATENT APPLICATION

(11) **EP 4 046 650 A1**
(43) Date of publication of application: **24.08.2022**
(21) Application number: 20876529.7
(22) Date of filing: 15.10.2020
(51) Int. Cl.: A61K 39/00, A61K 39/395, A61P 5/00, A61P 5/02, A61P 5/06, A61P 15/00, A61P 35/00, A61P 37/04, C07K 7/00, C07K 16/00, A61K 9/10, A61K 9/70, A61K 47/26, A61K 47/32, A61K 47/44, A61K 47/69, A61K 38/08, A61K 38/12, A61K 38/16, A61K 38/22

(54) **TRANSDERMAL ABSORPTION-TYPE PATCH**

(30) Priority: 16.10.2019 JP 2019189199; 26.03.2020 JP 2020056321; 24.08.2020 JP 2020140563
(71) Applicant: Kyushu University, National University Corporation, Nishi-ku Fukuoka-shi Fukuoka 819-0395 (JP)
(72) Inventor: GOTO Masahiro, Fukuoka-shi, Fukuoka 819-0395 (JP); KONG Qingliang, Fukuoka-shi, Fukuoka 819-0395 (JP); ISHIHAMA Kohei, Fukuoka-shi, Fukuoka 819-0395 (JP); KOZAKA Shuto, Fukuoka-shi, Fukuoka 819-0395 (JP)
(74) Representative: Epping - Hermann - Fischer
(86) International application number: PCT/JP2020/038883
(87) International publication number: WO 2021/075491

(57) **Abstract**

A transdermal absorption-type patch (10) includes: a support material (1) and an adhesive layer (2) laminated on the support material (1). The adhesive layer (2) includes: a solid composite material (2a), the solid composite material (2a) being an active ingredient with a molecular weight of 800 or greater enclosed by a surfactant; an oil phase; and an adhesive agent, the adhesive agent containing an acrylic elastomer. The content of the acrylic elastomer is 30% to 70% by mass based on the total mass of the acrylic elastomer and the oil phase. The composite material (2a) forms a solid-in-oil type particle dispersed in the oil phase.

SEQUENCE LISTING

<110>  Kyushu University, National University Corporation

<120>  Transdermal absorption type patch

<130>  20F103-PCT

<150>  JP 2019-189199
<151>  2019-10-16

<150>  JP 2020-056321
<151>  2020-03-26

<150>  JP 2020-140563
<151>  2020-08-24

<160>  1

<170>  PatentIn version 3.5

<210>  1
<211>  13
<212>  PRT
<213>  Artificial Sequence

<220>
<223>  TRP

<400>  1

Lys Lys Lys Gly Ser Val Tyr Asp Phe Phe Val Trp Leu
1               5                   10

## Description

### Technical Field

The present disclosure relates to a transdermal absorption-type patch.

### Background Art

Skin structure includes a stratum corneum, which acts as a physicochemical barrier that can non-specifically prevents invasion of different infectious agents including bacteria and viruses. Accordingly, it is extremely difficult to allow drugs to permeate into the body through the skin. In particular, hydrophilic macromolecules with molecular weights greater than 800 such as proteins are known not to be absorbed through the skin by themselves.

The present inventors have previously been developed preparations for allowing hydrophilic compounds to permeate into the body through the skin, including a solid-in-oil (S/O) type liquid preparation for external use (see, for example, Patent Literature 1) and a transdermal immunizing agent (see, for example, Patent Literature 2).

### Citation List

### Patent Literature

Patent Literature 1: International Publication No. WO 2006/025583
Patent Literature 2: Unexamined Japanese Patent Application Publication No. 2008-179561
Patent Literature 3: Unexamined Japanese Patent Application Publication No. 2014-172840

### Summary of Invention

### Technical Problem

However, when administering such a liquid S/O type preparation for external use, a difficulty exists in prescribing the exact amount per dose. Accordingly, in order to facilitate practical use of such S/O type preparations in medical settings, there is a need to incorporate the S/O type preparation into a self-adhesive tape or a self-adhesive sheet with release liner. Moreover, any transdermal absorption-type patch capable of allowing proteins with molecular weights greater than 800 to permeate into the skin had not previously been known.

Patent Literature 3 discloses a skin patch provided with an adhesive layer that includes an adhesive composition including an S/O type particle and an acrylic polymer, wherein the S/O type particle contains loxoprofen sodium dihydrate. The molecular weight of loxoprofen sodium dihydrate is approximately 300. Therefore, the composition of the adhesive layer disclosed in Patent Literature 3 is not optimal for S/O type particles containing macromolecules.

The present disclosure has been made considering the foregoing circumstances, and an objective of the present disclosure is to provide a transdermal absorption-type patch capable of enhancing skin penetrability of S/O type particles containing macromolecules.

### Solution to Problem

A transdermal absorption-type patch according to a first aspect of the present disclosure includes:
a support material; and
an adhesive layer laminated on the support material, wherein
the adhesive layer includes:
   a solid composite material, the solid composite material being an active ingredient with a molecular weight of 800 or greater enclosed by a surfactant;
   an oil phase; and
   an adhesive agent containing an acrylic elastomer,
the content of the acrylic elastomer is 30% to 70% by mass based on the total mass of the acrylic elastomer and the oil phase, and
the composite material forms a solid-in-oil type particle dispersed in the oil phase.

In the case of foregoing, the content of the acrylic elastomer may be 40% to 50% by mass based on the total mass of the acrylic elastomer and the oil phase.

The support material may include an acrylic resin.

The transdermal absorption-type patch according to the first aspect of the present disclosure may further include:
a liner layer on the adhesive layer covering the adhesive layer.

The active ingredient may be a protein having a molecular weight of greater than 10,000.

The active ingredient may be a protein having a molecular weight of 30,000 or greater.

The active ingredient may be a bioactive peptide, an antibody, or an antigen.

The transdermal absorption-type patch according to the first aspect of the present disclosure may further include:
a promoter layer disposed between the support material and the adhesive layer, the promoter layer containing a transdermal absorption promoter,
wherein the active ingredient may by an antigen.

The transdermal absorption-type patch according to the first aspect of the present disclosure may include an antigen as the active ingredient,
wherein the transdermal absorption-type patch may be used as a vaccine.

The active ingredient may be leuprorelin, octreotide or a salt thereof.

### Advantageous Effects of Invention

According to the present disclosure, the skin penetrability of S/O type particles containing macromolecules can be enhanced.

### Brief Description of Drawings

FIG. 1 is a schematic cross-sectional view of a transdermal absorption-type patch according to Embodiment 1 of the present disclosure;
FIG. 2 is a schematic cross-sectional view of a transdermal absorption-type patch according to another Embodiment 1 of the present disclosure;
FIG. 3 is a schematic cross-sectional view of a transdermal absorption-type patch according to another Embodiment 1 of the present disclosure;
FIG. 4 is a schematic cross-sectional view of a transdermal absorption-type patch according to another Embodiment 1 of the present disclosure;
FIG. 5 is a schematic illustration showing a transdermal absorption agent according to Embodiment 2 of the present disclosure;
FIG. 6 is a schematic cross-sectional view of a transdermal absorption testing system employed in Test Example 1;
FIG. 7 is a bar graph showing the results of the transdermal absorption test performed in Test Example 1 using FITC-OVA-encapsulated S/O self-adhesive sheet;
FIG. 8 is a bar graph showing the results of the transdermal absorption test performed in Test Example 2 using FITC-IgG-encapsulated S/O self-adhesive sheet;
FIG. 9 is a bar graph showing the results of the transdermal absorption test performed in Test Example 2 using OCT-encapsulated S/O self-adhesive sheet;
FIG. 10 is a diagram showing the time schedule of the animal immunization test performed in Test Example 3;
FIG. 11 is a bar graph showing the results of the animal immunization test performed in Test Example 3 using OVA-encapsulated S/O self-adhesive sheet;
FIG. 12 is a bar graph showing the results of the animal immunization test performed in Test Example 4 using OVA-encapsulated S/O self-adhesive sheet;
FIG. 13 is a graph showing the particle size distribution of the leuprorelin-surfactant composite material in S/O type preparation prepared in Example 4;
FIG. 14 is a schematic cross-sectional view of a transdermal absorption testing system employed in Test Example 5;
FIG. 15 is a bar graph showing the results of the transdermal absorption test performed in Test Example 5 using S/O type preparation;
FIG. 16 is a bar graph showing the results of the transdermal absorption test performed in Test Example 5 using S/O type preparation;
FIG. 17 is a bar graph showing the results of the mouse administration test to performed in Test Example 6 using S/O type preparation;
FIG. 18 is a graph showing the particle size distribution of the octreotide acetate-surfactant composite material in S/O type preparation prepared to Example 6;
FIG. 19 is a schematic cross-sectional view of a transdermal absorption testing system employed in Test Example 7;
FIG. 20 is a bar graph showing the amounts of the S/O preparation and Comparative Example 1 penetrated through the skin in Testing Example 7;
FIG. 21A and FIG. 21B are graphs showing the time course of the plasma concentration of octreotide acetate measured in Test Example 8; where FIG. 21A shows the time course of octreotide acetate concentration from 0 to 24 hours; and FIG. 21B shows the time course of octreotide acetate concentration from 0 to 96 hours;
FIG. 22A and FIG. 22B are graphs showing the time course of the plasma concentration of octreotide acetate measured in Test Example 9; where FIG. 22A shows the time course of octreotide acetate concentration from 0 to 24 hours; and FIG. 22B shows the time course of octreotide acetate concentration from 0 to 96 hours;
FIG. 23 is a graph showing the blood glucose levels measured in Testing Example 9;
FIG. 24 is a bar graph showing the results of the transdermal absorption test performed in Test Example 10 using FITC-K-TRP-2-encapsulated S/O self-adhesive sheet;
FIG. 25 is a bar graph showing the results of the transdermal absorption test performed in Test Example 11 using FITC-OVA-encapsulated S/O self-adhesive sheet;
FIG. 26A, FIG. 26B and FIG. 26C is a diagram showing the time schedule of the animal immunization test performed in Test Example 12 and graphs showing the results using OVA-encapsulated S/O self-adhesive sheet and OVA-encapsulated MGO self-adhesive sheet; where FIG. 26A is the diagram showing the time schedule of animal immunization test; FIG. 26B is the bar graph showing the antibody titer on day 35; and FIG. 26C is the bar graph showing the antibody titer on day 56;
FIG. 27 is a bar graph showing the results of the transdermal absorption test performed in Test Example 13 using FITC-OVA-encapsulated MGO self-adhesive sheet;
FIG. 28 is a bar graph showing the results of the transdermal absorption test performed in Test Example 14 using FITC-OVA-encapsulated bilayer S/O self-adhesive sheet; and
FIG. 29A and FIG. 29B are diagrams showing the time schedule of the animal immunization test performed in Test Example 15 and a graph showing the results using FITC-OVA-encapsulated bilayer S/O self-adhesive sheet; where FIG. 29A is the diagram showing the time schedule of animal immunization test; and FIG. 29B is the bar graph showing the amount of anti-OVA antibody.

### Description of Embodiments

A transdermal absorption-type patch according to the embodiment of the present disclosure is described hereinafter in detail.

### Embodiment 1

### <Transdermal absorption-type patch>

A transdermal absorption-type patch according to the present embodiment includes a support material and an adhesive layer laminated on the support material. The adhesive layer includes a solid composite material (hereinafter referred to as "active ingredient-surfactant composite material"), the solid composite material being an active ingredient with a molecular weight of 800 or greater enclosed by a surfactant; an oil phase; and an adhesive agent. The composite material forms a solid-in-oil (S/O) type particle dispersed in the oil phase.

As shown in Examples later, the transdermal absorption-type patch includes an adhesive layer containing a solid-in-oil (S/O) type particle in which a solid composite material is dispersed in an oil phase. This configuration enables to allow an active ingredient with a molecular weight of 800 or greater, which usually cannot penetrate through the skin into the body, to penetrate through the skin into the body. Furthermore, such a skin patch dosage form makes it possible to administer an exact specified amount of the active ingredient per one dose. Furthermore, because such a skin patch dosage form can be secured on the skin, the active ingredient can permeate through the skin into the body more efficiently than that in liquid S/O type preparations, as shown in Example below.

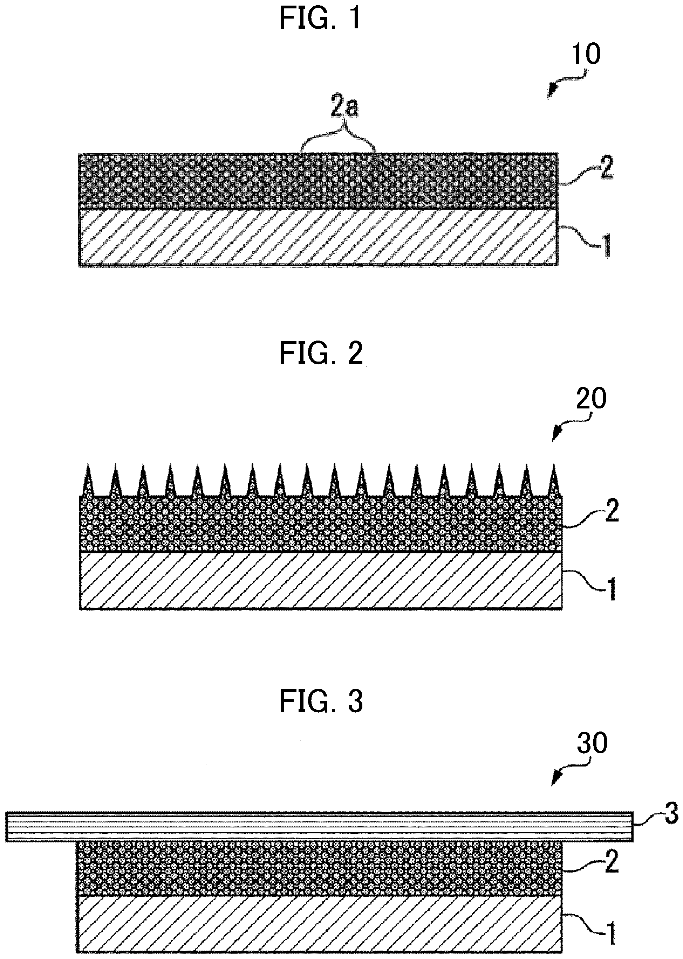

FIG. 1 is a schematic cross-sectional view of a transdermal absorption-type patch 10 as an example of a transdermal absorption-type patch according to the present embodiment. The transdermal absorption-type patch 10 includes a support material 1 and an adhesive layer 2. When viewed in plan view from the direction of the adhesive layer 2, the transdermal absorption-type patch 10 have a rectangular shape. The transdermal absorption-type patch 10 can be secured on the skin with the aid of the support material 1 and the adhesive layer 2.

The adhesive layer 2 is laminated on one side of the support material 1. The adhesive layer 2 includes a solid composite material 2a. The composite material 2a is constituted in such a way that an active ingredient with a molecular weight of 800 or greater is enclosed by a surfactant. In the adhesive layer 2, an oil phase is present without being lost by evaporation or a similar effect. Therefore, the composite material 2a is present as a dispersion in the oil phase in an admixture of all components of the adhesive layer 2.

FIG. 2 is a schematic cross-sectional view of a transdermal absorption-type patch 20 as another example of the transdermal absorption-type patch according to the present embodiment. In the description after FIG. 2, like elements already described in the previous figures are designated by like reference numerals as in the figures already described, and further detailed descriptions is omitted. The transdermal absorption-type patch 20 is identical to the transdermal absorption-type patch 10 as shown in FIG. 1 except that the adhesive layer 2 has one or more protrusions on the surface to be contacted with the skin. The one or more protrusions of the adhesive layer 2 on the surface to be contacted with the skin can facilitate a more effective permeation of the active ingredient through the skin into the body. The shape of the protrusion is not particularly limited, but the examples include a pyramidal shape and a conical shape. The size of each protrusion is not particularly limited, but from the viewpoint of enhancing the permeation effect of the active ingredient into the body, the height, width, or length may be on the order of micrometers, for example, from 1 µm or more to 1,000 µm or less.

FIG. 3 is a schematic cross-sectional view of a transdermal absorption-type patch 30 as another example of the transdermal absorption-type patch according to the present embodiment. The transdermal absorption-type patch 30 is identical to the transdermal absorption-type patch 10 as shown in FIG. 1 except that the adhesive layer 2 is covered with a liner layer 3 disposed on the adhesive layer 2. In other words, the transdermal absorption-type patch 30 includes a support material 1, an adhesive layer 2, and a liner layer 3, laminated in this order. By providing the liner layer 3, the transdermal absorption-type patch 30 can be stored until the transdermal absorption-type patch 30 is applied to the skin while protecting the adhesive layer 2.

The transdermal absorption-type patch according to the present embodiment is not particularly limited to those shown in FIGS. 1 to 3, but the configurations shown in FIGS. 1 to 3 may be altered in part or an additional configuration may be added thereto, to the extent that the advantageous effect of the transdermal absorption-type patch is not impaired.

For example, the transdermal absorption-type patches 10, 20, and 30 as shown in FIGS. 1 to 3 have rectangular shapes when viewed in plan view, but other shapes are possible. Examples of the planar shape of the transdermal absorption-type patches 10, 20, and 30 include, but not limited to, polygonal (including regular polygonal), semicircular, ellipsoidal, generally circular, and true circular shapes.

In the transdermal absorption-type patch 30 as shown in FIG. 3, by way of example, a release layer may be provided onto the liner layer 3 on the side to be contacted with the adhesive layer 2 so that the liner layer 3 can be easily peeled off from the surface of the adhesive layer 2. The release layer may be prepared by disposing a known release agent such as, for example, a silicone-based resin or a fluorine-based resin on the surface of the liner layer 3 to be contacted with the adhesive layer 2.

In the following, each component constituting the transdermal absorption-type patch is described in detail.

### [Adhesive layer]

The adhesive layer is formed from an adhesive composition comprising a solid composite material, an oil phase, and an adhesive agent. In the adhesive composition, the solid composite material forms an S/O type particle dispersed in the oil phase.

The thickness of the adhesive layer may be, for example, from 1 µm or more to 5,000 µm or less. When the thickness of the adhesive layer is within the above-described range, the adhesive layer can retain a more sufficient amount of the S/O type particle. As used herein the term "thickness of the adhesive layer" refers to the thickness of the entire adhesive layer. For example, if the adhesive layer is made up of a plurality of layers, the thickness of the adhesive layer refers to the total thickness of all layers making up the adhesive layer.

### (S/O Type particle)

Describing the S/O type particle, the solid phase (S) is a solid material constituting the dispersed phase and the oil phase (O) is an oily base material constituting the continuous phase. The solid phase contains an active ingredient. The active ingredient is not limited to a particular substance as long as it exerts some function in a living organism and also has a molecular weight of 800 or greater. The active ingredient is preferably hydrophilic. Examples of the active ingredient include proteins, antibodies, antigens, leuprorelin, octreotide, or salts thereof.

Examples of the hydrophilic active ingredient include antigens, antidementia drugs, oligonucleotide therapeutics, anti-biotic preparations, anti-neoplastic drugs, anti-fungal drugs, anti-bacterial drugs, antibody preparations, enzyme preparations, antiestrogen drugs, immunosuppressive drugs, schizophrenia drugs, antiepileptic drugs, anti-depressant drugs, anti-Parkinson drugs, anti-allergic drugs, anticancer drugs, anti-diabetic drugs, anti-hypertensive drugs, osteoporosis drugs, ED (Erectile Dysfunction) drugs, skin disease drugs, local anesthetics, and pharmaceutically acceptable salts thereof. The pharmaceutically acceptable salt of the active ingredient is not particularly limited, but either an acidic salt or a basic salt may be employed. More specifically, the examples of the hydrophilic active ingredient include octreotide acetate, leuprorelin acetate, teriparatide acetate, glatiramer acetate, insulin, growth hormone (somatropin), somatomedin, glucagon, urokinase, pro-urokinase, erythropoietin, G-CSF, IL-2, INF (alpha, beta, gamma), tPA, Factor VIII, antibody, and recombinants thereof, and pharmaceutically acceptable analogs thereof.

The protein as the active ingredient contained in the S/O type particle has a molecular weight of 800 or greater, preferably greater than 10,000, and more preferably 30,000 or greater. The upper limit of the molecular weight is not particularly limited, but may be, for example, 2,000,000 or lower, 1,000,000 or lower, 500,000 or lower, or 300,000 or lower. By forming the S/O type particle in which the composite material is dispersed in the oil phase in the adhesive layer, the transdermal absorption-type patch is enabled to allow the protein having a molecular weight within the above-described range to permeate into the body. As used herein, the term "molecular weight" refers to weight average molecular weight, which may be determined, for example, by gel permeation chromatography (GPC).

Examples of such proteins include bioactive peptides, antibodies, and antigens. Examples of the bioactive peptide include hormones such as insulin and neuropeptides.

The antigen is not limited to a particular substance as long as it is soluble or dispersible in an aqueous phase and also is able to elicit immune response and to induce immunogenicity. Antigens used for purposes such as conventional immunization, vaccine antigens, immunogenic substances, and cancer antigens may be used. Specifically, the examples include proteins and peptides such as biologically active substances (for example, lysozyme derived from chicken egg white and peptides containing an antigenic region associated with Japanese cedar pollinosis), substances haptenized to a protein or a peptide, live or inactivated (killed) bacteria and viruses (whole) or their partially degraded products, inactivated (killed) cancer cells (whole) or their partially degraded products, and nucleic acids. In addition, substances that do not have antigenicity (for example, carbohydrates, lipids, and inorganic substances) may also be used as antigens by imparting antigenicity to the substances by means such as haptenization. The antigen may be of animal or plant origin. For example, the antigen may be those for poultry and livestock or for pets, or may be those for cattle, chicken, or pig, or may be those derived from arthropods and insects such as ticks. Antigens may also be of food origin.

These antigens may be in a form of a single molecule or in a form of a hydrophilic water-soluble nanocarrier such as a nanogel (cholesterol-modified pullulan) encapsulating an antigen such as a hydrophobic antigen. In addition, these antigens may also be used as an admixture with a water-soluble hydrophilic adjuvant such as a CpG oligodeoxynucleotide as described below. Preferably, the above-described antigen is a hydrophilic antigen.

In addition, the examples of proteins other than those mentioned above include quasi-drug ingredients such as collagen and growth factors including epidermal growth factor, insulin-like growth factor, fibroblast growth factor, and brain-derived neurotrophic factor as well as cosmetic ingredients. The S/O type particle may contain any of these proteins alone or a combination of two or more of those.

In the S/O type particle, the solid phase is typically formed as a composite material. The method for producing the composite material is not particularly limited, and various methods used for producing the composite material may be applicable. Typically, in order to produce the composite material, a solution of an active ingredient is emulsified and dispersed in an organic solvent containing a surfactant to form a W/O type emulsion, and thereafter the resulting W/O type emulsion is lyophilized by a conventional technique, to thereby obtain the composite material. The organic solvent is not particularly limited as long as it can dissolve lipophilic surfactants and also can be removed by lyophilization. However, lower alcohols such as ethanol and low boiling point hydrocarbons such as hexane are normally used. The emulsification and the dispersion may be performed by using conventional apparatus such as stirrers, high-speed high-shear mixers (such as propeller mixers), colloid mills, homogenizers, flow jet mixers, ultrasonic emulsifiers, and vacuum emulsifiers. The particle size of the dispersed droplets may be controlled by adjusting the mixing intensity (= mixing power × mixing time). A detailed description of such techniques may be referred to Unexamined Japanese Patent Application Publication No. 2004-43355 (Reference Literature 1).

The surfactants employed for producing the composite material are those acceptable for addition to pharmaceutical preparations, preferably to pharmaceutical preparations for transdermal delivery and have an ability to stably disperse the solid phase in the oil phase. Examples of such surfactant include nonionic surfactants, anionic surfactants, cationic surfactants, amphoteric surfactants, and bile salts.

Examples of the surfactant suitable for producing the composite material include a lipophilic (hydrophobic) nonionic surfactant having an HLB (Hydrophile-Lipophile Balance) value 10 or lower. The HLB of the nonionic surfactant is preferably 8 or lower, more preferably 5 or lower, and especially preferably 3 or lower. The nonionic surfactant is preferably an ester compound derived from an unsaturated fatty acid such as erucic acid or oleic acid. Examples of the lipophilic nonionic surfactant include the following esters having a higher degree of esterification (in other words, containing di-, tri-, and polyesters in higher proportions relative to monoesters): sucrose fatty acid esters (sucrose stearic acid esters, sucrose palmitic acid esters, sucrose myristic acid esters, sucrose oleic acid esters, sucrose lauric acid esters, sucrose erucic acid esters, sucrose esters with mixed fatty acids), polyglycerin condensed ricinoleic acid esters, decaglycerin esters, glycerin fatty acid esters, polyglycerin fatty acid esters, polyoxyethylene glycerin fatty acid esters, sorbitan fatty acid ester, polyoxyethylene sorbit fatty acid esters, and polyoxyethylene castor oil and hardened castor oil. Suitable nonionic surfactants are sucrose lauric acid esters. The surfactant may be used alone or used as a mixture of two or more surfactants.

From the standpoint that the transdermal absorption-type patch is exclusively employed for transdermal delivery, the surfactant preferably has an additional effect such as absorption promotion, irritation relief, or moisturization.

The composite material is dispersed in the oil phase to form the S/O type particle preferably prior to being admixed with other components such as an adhesive agent to prepare the adhesive composition. The concentration of the composite material in the oil phase is not particularly limited as long as the composite material can be dispersed therein. However, it may be from 0.6% by mass or higher to 60% by mass or lower relative to the total mass of the dispersion or suspension.

The oil phase may be in a form of liquid or may be in a form of flowable or spreadable solid. The base material of the oil phase is not particularly limited as long as it is acceptable for use in pharmaceutical preparations, preferably in pharmaceutical preparations for transdermal delivery. Any oil, which is liquid at room temperature (25°C), or any fat, which is solid at room temperature, may be used as the oil phase. The origin of the raw materials of the base material is also not particularly limited. The raw materials may be, for example, either of natural or synthetic origin, and may be of vegetable origin (for example, soybean oil, cottonseed oil, rapeseed oil, sesame oil, corn oil, peanut oil, safflower oil, sunflower oil, olive oil, rapeseed oil, perilla oil, fennel oil, cocoa oil, cinnamon oil, mentha oil, bergamot oil) or of animal origin (beef tallow, pork oil, fish oil). In addition, neutral lipids (for example, glycerides, triolein, trilinolein, tripalmitin, tristearin, trimyristin, triarachidonin), synthetic lipids, sterol derivatives (for example, cholesteryl oleate, cholesteryl linoleate, cholesteryl myristate, cholesteryl palmitate, cholesteryl arachidate), long-chain fatty acid esters (for example, isopropyl myristate, octyldodecyl myristate, cetyl myristate, ethyl oleate, ethyl linoleate, isopropyl linoleate, isopropyl palmitate, butyl stearate), carboxylic acid esters (ethyl lactate, cetyl lactate, triethyl citrate, diisopropyl adipate, diethyl sebacate, diisopropyl sebacate, cetyl 2-ethylhexanoate), hydrocarbons (for example, petroleum jelly, paraffin squalane, vegetable squalane), and silicones may also be used. The oily base material may be used alone or as a mixture of two or more of these materials.

Triglycerides or dietary oils containing a triglyceride as a main component may also be used as the oil phase. Soybean oil is preferred, and highly purified soybean oil is more preferred for practical reasons. Neutral lipids or long-chain fatty acid esters may also be used as the oil phase, with long-chain fatty acid esters being more preferred, and isopropyl myristate being even more preferred.

### (Adhesive agent)

The adhesive agent may be any material that is not toxic to living organisms. The specific type of the adhesive agent may be determined appropriately in accordance with the support material.

Examples of the adhesive agent include, but are not limited to, adhesive agents made of synthetic compounds or adhesive agents made of naturally occurring compounds. Examples of the adhesive agent made of synthetic compounds include urethane-based elastomers, acrylic elastomers, olefinic elastomers, silicone-based elastomers, rubber-based adhesive agent, calcium phosphate-based adhesives, and resinbased cements. Examples of the rubber-based adhesive agent include styrenic elastomers, polyisobutylene (PIB), polyisoprene, polybutadiene, and natural rubber. Among these adhesive agents, silicone-based elastomers, rubber-based adhesives, or acrylic elastomers are preferred, with acrylic elastomers being more preferred from a viewpoint of enhancing permeability.

Suitably, the adhesive agent contained in the adhesive layer includes an acrylic elastomer. The acrylic elastomer preferably includes a structural unit derived from an alkyl (meth)acrylate having an alkyl group with 6 to 14 carbon atoms in the ester portion. More specifically, the examples of the alkyl (meth)acrylate include hexyl (meth)acrylate, n-octyl (meth)acrylate, isooctyl (meth)acrylate, 2-ethylhexyl (meth)acrylate, dodecyl (meth)acrylate, decyl (meth)acrylate, isodecyl (meth)acrylate, lauryl (meth)acrylate, and myristyl (meth)acrylate. Any of these alkyl (meth)acrylates may be used alone or in combination of two or more.

The acrylic elastomer may further comprise, in addition to the structural unit derived from the above-described (meth)acrylic acid alkyl ester, a structural unit derived from a vinyl monomer having a reactive functional group. This facilitates the reaction with other structural units, especially with a crosslinking agent, if the crosslinking agent is present. Examples of the reactive functional group include hydroxyl group, amino group, carboxy group, and thiol group. Only one type of such reactive functional groups may be present, or two or more types of such reactive functional groups may be present. Among these reactive functional groups, hydroxyl group, amino group, or carboxy group is preferred. By incorporating a structural unit derived from a vinyl monomer having such a reactive functional group into the acrylic elastomer, it becomes possible to control the cohesive force of the adhesive composition to form the adhesive layer.

Examples of the above-described vinyl monomer having a reactive functional group include hydroxyl group-containing (meth)acrylates such as hydroxyethyl (meth)acrylate, hydroxypropyl (meth)acrylate, and hydroxybutyl (meth)acrylate; 1,4-di(meth)acryloxyethyl pyromellitic acid, 4-(meth)acryloxyethyl trimellitic acid, carboxy group-containing (meth)acrylates such as N-(meth)acryloyl-p-aminobenzoic acid, 2-(meth)acryloyloxybenzoic acid, N-(meth)acryloyl-5-aminosalicylic acid, acrylic acid, and methacrylic acid; primary or secondary amino group-containing (meth)acrylates such as aminoethyl (meth)acrylate, ethylaminoethyl (meth)acrylate, aminopropyl (meth)acrylate, and ethylaminopropyl (meth)acrylate; and thiol group-containing (meth)acrylates such as 2-(methylthio)ethyl methacrylate. Any of these vinyl monomers may be used alone or in combination of two or more.

In addition to the structural unit derived from the above-described (meth)acrylic acid alkyl ester, the acrylic elastomer may further include a structural unit derived from a monomer having a plurality of polymerizable unsaturated groups. In other words, the acrylic elastomer may be a copolymer prepared from a monomer composition comprising an alkyl (meth)acrylate having an alkyl group with 6 to 14 carbon atoms in its ester portion and a monomer having a plurality of polymerizable unsaturated groups.

Examples of the above-described monomer having a plurality of polymerizable unsaturated groups include alkylene glycols such as (poly)ethylene glycol, (poly)propylene glycol, (poly)butylene glycol, (poly)pentamethylene glycol, and (poly)hexamethylene glycol; trimethylolpropane; glycerine polyoxyethylene glycol; di- or tri-(meth)acrylic acid esters with polyols such as polyoxypropylene glycol and (poly)glycerol; diallyl phthalate; and divinyl benzene. Any of these monomers may be used alone or in combination of two or more. Among the above-described monomers, alkylene glycol diacrylates are preferred because they exhibit higher reactivity with other monomers and can suitably enhance cohesive strength of the acrylic elastomer.

The method for preparing the acrylic elastomer is not particularly limited. The acrylic elastomer may be prepared by polymerizing starting monomers corresponding to each of the structural units by use of a polymerization technique such as solution polymerization. Alternatively, a commercially available product of acrylic elastomers may be used.

Examples of commercially available acrylic elastomers include "DURO TAK 87-4098", "DURO TAK 87-2052", "DURO TAK 87-9301", "DURO TAK 87-235A", "DURO TAK 87-4287", and "DURO TAK 87-2516" manufactured by Henkel AG & Co. KGaA and "MAS 811B", "MAS 683", "MAS43", "MASCOS10", and "HiPAS10" manufactured by CosMED Pharmaceutical Co. Ltd.

If a styrenic elastomer is used, a styrenic block copolymer may be preferred. Specific examples of the styrenic block copolymer include styrene-butadiene block copolymers, styrene-butadiene-styrene block copolymers, styrene-isoprene block copolymers, styrene-isoprene-styrene block copolymers (SIS), styrene-ethylene/butylene block copolymer, styrene-ethylene/butylene-styrene block copolymers, styreneethylene/propylene block copolymers, styrene-ethylene/propylene-styrene block copolymers, styrene-isobutylene block copolymer, and styrene-isobutylene-styrene block copolymers. As used in the above description, the term "ethylene/butylene" refers to a copolymer block of ethylene and butylene, and the term "ethylene/propylene" refers to a copolymer block of ethylene and propylene. Any of these styrenic block copolymers may be used alone or in combination of two or more.

When the styrene-isoprene-styrene block copolymer and the styrene-isoprene block copolymer are used, the respective copolymers may be prepared by using corresponding preparation methods known per se. Alternatively, the respective commercially available products of the styrene-isoprene-styrene block copolymer and the styrene-isoprene block copolymer may be used. Alternatively, mixtures of the styrene-isoprene-styrene block copolymer and the styrene-isoprene block copolymer are also commercially available, and such commercially available mixtures of the styrene-isoprene-styrene block copolymer and the styrene-isoprene block copolymer may suitably be used.

Examples of the commercially available product of the styrenic block copolymer include "KRATON D1163", "KRATON D1113", and "KRATON D1119" manufactured by KRATON POLYMERS, "JSR SIS5229", "JSR SIS5403", "JSR SIS5505" manufactured by JSR Corporation, and "Quintac 3421", "Quintac 3433N", "Quintac 3520", "Quintac 3450", and "Quintac 3270" manufactured by Zeon Corporation.

Examples of the adhesive agent made from a naturally occurring compound include fibrin glue and gelatin glue.

### (Other component)

The adhesive composition for forming the adhesive layer may further comprise other components such as a crosslinking agent and a transdermal absorption promoter.

Examples of the crosslinking agent include multi-functional amino compounds, multi-functional isocyanate compounds, multi-functional epoxy compounds, multi-functional aziridine compounds, multi-functional oxazoline compounds, and multi-functional metal compounds. Any of these crosslinking agents may be used alone or in combination of two or more.

The transdermal absorption promoter may be any compound that was previously shown to have an absorption promoting effect into the skin. Examples of such compounds include fatty acids with 6 to 20 carbon atoms, aliphatic alcohols, fatty acid amides, fatty acid ethers, aromatic organic acids, aromatic alcohols, aromatic organic acid esters or ethers, lactic acid esters, acetic acid esters, monoterpenic compounds, sesquiterpenic compounds, glycerin fatty acid esters, propylene glycol fatty acid esters, sorbitan fatty acid esters, polysorbates, polyethylene glycol fatty acid esters, polyoxyethylene hydrogenated castor oils, polyoxyethylene alkyl ethers, sucrose fatty acid esters, and vegetable oils. Preferably, the transdermal absorption promoter contains glyceryl monooleate (MGO) or similar compounds, and especially preferably glyceryl oleate such as MGOL-70 that enhances skin permeability of the active ingredient by temporarily disrupting the lamellar structure of the stratum corneum.

Additionally, when the S/O type particle contains an antigen, the adhesive composition may further contain a hydrophobic adjuvant. The hydrophobic adjuvant is not particularly limited if it is an adjuvant normally used for vaccines, but the hydrophobic adjuvant may be, for example, a ligand for innate immune receptor. As used herein, the term "ligand" refers to a substance that specifically binds to a receptor. In particular, a substance that specifically binds to a receptor to exert various physiological effects may be used. Such substances are also called as "agonists."

Examples of the innate immune receptor include toll-like receptors (TLRs), RIG-I-like receptors (RLRs), NOD-like receptors (NLRs), and C-type lectin receptors (CLRs). Among these ligands, ligands for TLRs (hereinafter sometimes referred to as "TLR ligand") or ligands for CLRs (hereinafter sometimes referred to as "CLR ligand") are preferred, and ligands for TLRs on the cell membrane of antigen presenting cells are more preferred because they are known to significantly contribute to activation of antigen presenting cells.

The TLR ligand may be appropriately selected from ligands that interact with at least one TLR selected from the group consisting of TLR-2, TLR-3, TLR-4, TLR-5, TLR-6, TLR-7, TLR-8, and TLR-9, for example. Examples of the TLR-2 ligand include Pam3 CSK4. Examples of the TLR-3 ligand include poly ICLC, polyinosinic:polycytidylic acid (poly I:C). Examples of the TLR-4 ligand include R-type lipopolysaccharide, S-type lipopolysaccharide, Paclitaxel, lipid A, and monophosphoryl lipid A. Examples of the TLR-5 ligand include Flagellin.

Examples of the ligand of TLR-2 and TLR-6 include MALP-2. Examples of the ligand of TLR-7 and TLR-8 include resiquimod (R848), imiquimod (R837), gardiquimod, and loxoribine. Examples of the TLR-9 ligand include CpG oligodeoxynucleotides. Among these ligands, resiquimod (R848) is preferred as the hydrophobic adjuvant because its safety and immune activating effect have previously confirmed in humans and mice.

The CLR ligand may be appropriately selected from ligands that interact with at least one CLR selected from the group consisting of macrophage inducible c-type lectin (Mincle) receptor, macrophage c-type lectin (MCL) receptor, dendritic cellassociated c-type lectin-2 (Dectin-2) receptor, and Dendritic Cell Immunoactivating Receptor (DCAR).

Examples of the Mincle receptor ligand and the MCL receptor ligand include trehalose dimycolate. Examples of the Dectin-2 receptor ligand include lipoarabinomannan. Examples of the DCAR ligand include phosphatidylinositol mannosides.

The adhesive composition for forming the adhesive agent may be prepared by mixing each component constituting the composition. In the preparation of the adhesive composition, an S/O type particle (a solution or dispersion of the composite material dispersed in the oil phase) (solid content: from 0.6% by mass or more to 60% by mass or less) is mixed with an adhesive agent (solid content: from 20% by mass or more to 80% by mass or less) in a mixing ratio of 5:1 to 1:5, preferably 3:1 to 1:3, more preferably 2:1 to 1:2, further preferably 1.25:1 to 1:1.25, and especially preferably 1:1.

When the adhesive agent contained in the adhesive layer includes an acrylic elastomer, the content of the acrylic elastomer in the adhesive layer is suitably 30% to 70% by mass, and preferably 40% to 50% by mass based on the total mass of the acrylic elastomer and the oil phase.

### [Support material]

Preferably, the support material is flexible (soft), is able to follow curved surfaces when applied to the skin, and can be easily cut or punched during processing.

Such a support material is not particularly limited, but may be, for example, plastic films, woven fabrics, knitted fabrics, and nonwoven fabrics made of resins including polyesters such as polyethylene terephthalate (PET), polybutylene terephthalate (PBT), and polyethylene naphthalate (PEN); polyolefins such as polyethylene (PE) and polypropylene (PP); polyarylate; polyurethane; polycarbonate; polyamide; polyimide; ethylene vinyl acetate copolymer; polyvinyl chloride; polytetrafluoroethylene; silicone; polysulfone; nylon; polylactic acid; rayon; and acrylic resins. In addition, a laminated sheet or a similar material consisting of two or more layers in which one of the two or more layers is a plastic film and the other layers are plastic films, woven fabrics, knitted fabrics, nonwoven fabrics, papers, or the like may be used. Among these materials, plastic films made of acrylic resins are preferred.

The thickness of the support material may be, for example, from 1 µm or more to 5,000 µm or less. As used herein the term "thickness of the support material" refers to the thickness of the entire support material. For example, if the support material is made up of a plurality of layers, the thickness of the support material refers to the total thickness of all layers making up of the support material.

### [Liner layer]

Examples of the liner layer that may be used include laminated papers such as polyethylene laminated paper and polypropylene laminated paper; and films of synthetic resins including polyester-based films such as polyethylene terephthalate film and polyolefin-based films such as polyethylene film and polypropylene film. In addition, one or both sides of the liner layer may be subjected to release treatment with a release agent such as a silicone resin or a fluorine resin as required.

The thickness of the liner layer maybe, for example, from 10 µm or more to 1,000 µm or less. As used herein the term "thickness of the liner layer" refers to the thickness of the entire liner layer. For example, if the liner layer is made up of a plurality of layers, the thickness of the liner layer refers to the total thickness of all layers making up of the liner layer.

### <Method for producing transdermal absorption-type patch>

The transdermal absorption-type patch 10 shown in FIG. 1 or the transdermal absorption-type patch 20 shown in FIG. 2 may be produced, for example, by applying an adhesive composition to the support material 1 and drying the adhesive composition to form the adhesive layer 2.

The transdermal absorption-type patch 30 shown in FIG. 3 may be produced by one of the following two procedures:
(A) This procedure is carried out as follows: the adhesive composition is applied to the support material 1, followed by drying the adhesive composition to form the adhesive layer 2, and subsequently, the liner layer 3 is attached to the surface of the adhesive layer 2 opposite to the surface in contact with the support material 1, yielding the transdermal absorption-type patch 30;
(B) This procedure is carried out as follows: the adhesive composition is applied to the liner layer 3, followed by drying the adhesive composition to form the adhesive layer 2, and subsequently, the support material 1 is attached to the surface of the adhesive layer 2 opposite to the surface in contact with the liner layer 3, yielding the transdermal absorption-type patch 30.

The adhesive composition may be prepared in advance by using the procedure described above in the "Adhesive layer" section. The content of the active ingredient in the transdermal absorption-type patch may be appropriately adjusted in accordance with the therapeutically effective amount of the active ingredient by adjusting the content of the S/O type particle.

### <Applications>

The transdermal absorption-type patch according to the present embodiment may be used, for example, as a vaccine when the active ingredient contained in the S/O type particle is an antigen. In other words, in one embodiment, a transdermal absorption-type patch for inducing immunity (for vaccination) includes a support material and an adhesive layer laminated thereon, wherein the adhesive layer includes a solid composite material, the solid composite material being an antigen with a molecular weight of 800 or greater enclosed by a surfactant; an oil phase; and an adhesive agent, wherein the solid composite material forms S/O type particles dispersed in the oil phase.

If the active ingredient contained in the S/O type particles is an antibody, for example, the transdermal absorption-type patch may be used as pharmaceutical composition (antibody pharmaceuticals) for treating various diseases according to the type of the antibody. Specifically, when the active ingredient contained in the S/O type particle is a PD-1 antibody, the transdermal absorption-type patch may be used as an anticancer agent.

If the active ingredient contained in the S/O type particle is a physiologically active peptide, for example, the transdermal absorption-type patch may be used as pharmaceutical compositions for treating various diseases. Specifically, when the active ingredient contained in the S/O type particle is insulin, the transdermal absorption-type patch may be used as a pharmaceutical composition for treating diabetes mellitus.

When the active ingredient contained in the S/O type particles is a collagen, the transdermal absorption-type patch may be used as a quasi-drug composition or as a cosmetic composition.

The subject to be treated with the transdermal absorption-type patch is preferably a vertebrate animal, and more preferably a mammalian animal. Examples of the mammalian animal include, but not limited to, human, chimpanzee, and other primates; domestic animals; pet animals; and laboratory animals such as dogs, cats, rabbits, horses, sheep, goats, cattle, pigs, rats (including nude rats), mice (including nude mice and skid mice), and guinea pigs. Among these mammalian animals, human is preferred.

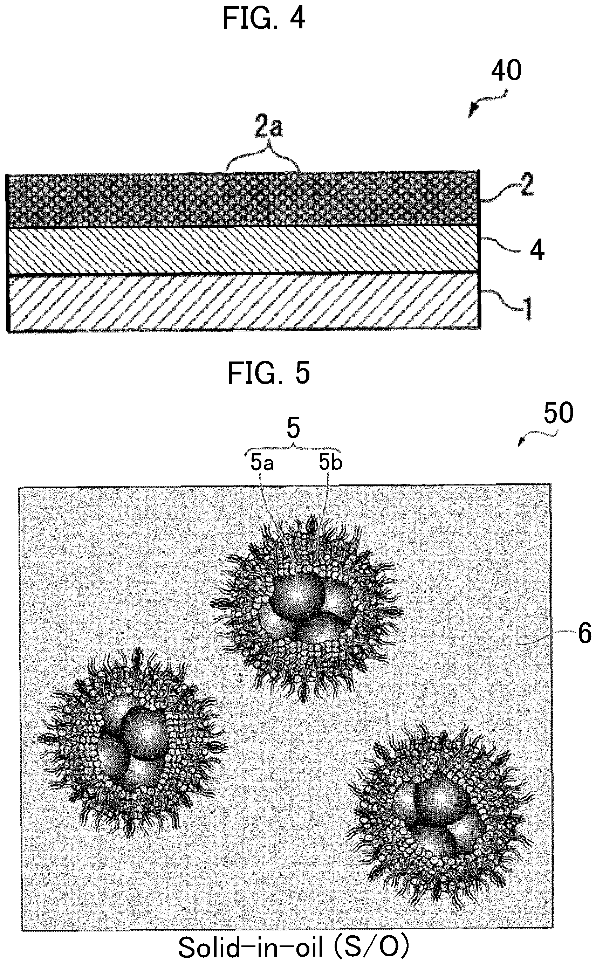

When the active ingredient is an antigen, the transdermal absorption-type patch according to a preferred embodiment may further comprise a promoter layer disposed between the support material and the adhesive layer, the promoter layer containing a transdermal absorption promoter. FIG. 4 is a schematic cross-sectional view of a transdermal absorption-type patch 40 as an example of the transdermal absorption-type patch. The transdermal absorption-type patch 40 includes a promoter layer 4 containing a transdermal absorption promoter. The promoter layer 4 is interposed between the support material 1 and the adhesive layer 2.

The transdermal absorption-type patch 40 may be produced, for example, by applying a transdermal absorption promoter composition containing a transdermal absorption promoter to the support material 1, followed by drying the transdermal absorption promoter composition to form the promoter layer 4, and subsequently applying an adhesive composition to the resultant promoter layer 4, followed by drying the adhesive composition to form the adhesive layer 2. The promoter layer 4 is formed from a transdermal absorption promoter composition containing a transdermal absorption promoter. The transdermal absorption promoter in the transdermal absorption promoter composition may be in a dissolved state in a solvent. The solvent is not particularly limited as long as it can dissolve the transdermal absorption promoter, but an oil phase is preferred. As the oil phase, a similar material to those recited above with reference to the oil phase may be used. Suitably, the same oil phase contained in the transdermal absorption promoter composition is used as the oil phase contained in the adhesive composition. The content of the transdermal absorption promoter in the transdermal absorption promoter composition is, for example, 1 to 30% by mass, 3 to 25% by mass, 5 to 20% by mass, or 8 to 15% by mass, with 10% by mass being preferred.

The transdermal absorption promoter composition may comprise the adhesive agent described above. When the transdermal absorption promoter composition includes the adhesive agent, the mixing ratio of the solvent containing the transdermal absorption promoter agent to the adhesive agent is not particularly limited, but, for example, 1:1.

Analogous to the transdermal absorption-type patch 30, the adhesive layer 2 in the transdermal absorption-type patch 40 may be covered with a liner layer 3 disposed on the adhesive layer 2. The transdermal absorption-type patch 40 having this configuration may be produced by applying an absorption promoter composition containing a transdermal absorption promoter to the support material 1, followed by drying the absorption promoter composition to form the promoter layer 4, and subsequently applying an adhesive composition to the resultant promoter layer 4, followed by drying the adhesive composition to form the adhesive layer 2, and subsequently attaching the liner layer 3 to the surface of the resultant adhesive layer 2 opposite to the surface in contact with the promoter layer 4. Alternatively, the transdermal absorption-type patch 40 may be produced by applying an adhesive composition to the liner layer 3, followed by drying the adhesive composition to form the adhesive layer 2, and subsequently applying a transdermal absorption promoter composition to the resultant adhesive layer 2, followed by drying the transdermal absorption promoter composition to form the promoter layer 4, and subsequently attaching the support material 1 to the surface of the resultant promoter layer 4 opposite to the surface in contact with the adhesive layer 2.

With the aid of the promoter layer containing the transdermal absorption promoter incorporated into the transdermal absorption-type patch between the support material and the adhesive layer, the antigen is enabled to allow to permeate more efficiently through the skin into the body and the sustained release properties thereof is also improved. As a result of this, as shown in Example below, the antibody titer can be maintained in a certain level overtime after immunization with antigen.

### Embodiment 2

In the following, the transdermal absorption agent according to Embodiment 2 is described. The transdermal absorption agent according to the present embodiment contains leuprorelin as the active ingredient.

Leuprorelin is an agonist for gonadotropin-releasing hormone (GnRH) receptor that acts to suppress secretion of gonadotropic hormones, namely, luteinizing hormone (LH) and follicle stimulating hormone (FSH) in the pituitary gland. In 1992, a sustained release preparation of leuprorelin was approved for treatment of prostate cancer. Thereafter, the sustained release preparation of leuprorelin was approved for indications including estrogen-dependent diseases (such as endometriosis and uterine fibroids), central precocious puberty, and premenopausal breast cancer. Leuprorelin is a hydrophilic compound, and is currently only administered in injectable form.

Drug administration by injection has been indicated to cause a problem of side effect such as anaphylaxis due to initial rise in blood drug concentration. From the viewpoint to suppress side effects and to extend the duration of drug efficacy, it would be desirable to have a dosage form in a transdermal absorption agent. However, skin penetrability of leuprorelin is extremely low because leuprorelin is a hydrophilic compound. Therefore, the drug efficacy of the leuprorelin cannot sufficiently be exerted if it is formulated in a transdermal absorption agent. Accordingly, there is a need to develop a transdermal absorption agent containing leuprorelin that exhibits an improved skin penetrability and a prolonged duration of the drug effect.

### <Transdermal absorption agent>

The transdermal absorption agent according to the present embodiment includes a solid leuprorelin-surfactant composite material (hereinafter also simply referred to as "composite material") and an oil phase, which solid leuprorelin-surfactant composite material being leuprorelin or a salt thereof (hereinafter collectively referred to as "leuprorelin") enclosed by a surfactant. The composite material forms a solid-in-oil type particle dispersed in the oil phase.

By virtue of the composite material forming the S/O type particle dispersed in the oil phase, the transdermal absorption agent is enabled to allow leuprorelin, which normally would not penetrate through the skin, to permeate through the skin into the body as shown in Example below. Furthermore, the transdermal absorption agent has better sustained release properties than conventional injectable forms as shown in Example below.

FIG. 5 is a schematic illustration showing a transdermal absorption agent 50 as an example of the transdermal absorption agent according to the present embodiment. The composite material 5 is composed such that leuprorelin 5a is associated with the hydrophilic portion of a surfactant 5b whereby the leuprorelin 5a is enclosed by the surfactant 5b. In the transdermal absorption agent 50, the composite material 5 is dispersed in the oil phase 6, forming an S/O type particle.

The transdermal absorption agent according to the present embodiment is not particularly limited to that shown in FIG. 5, but a part of the configuration shown in FIG. 5 may be altered or omitted, or further configuration may be added to the configuration described hereinbefore, to the extent that the advantageous effect of the transdermal absorption agent is not impaired.

For example, in the transdermal absorption agent 50 shown in FIG. 5, a transdermal absorption promoter may be dispersed in the oil phase 6 for the purpose of enhancing the permeability into the skin.

In the following, each component of the transdermal absorption agent is described in detail.

### [Solid leuprorelin-surfactant composite material]

The solid composite material includes leuprorelin and a surfactant. The composite material may consist exclusively of leuprorelin and a surfactant to prevent the particle from growing too large, or alternatively, the composite material may further contain a stabilizing agent as described below.

As regards to the average particle size of the composite material dispersed in the oil phase, it is sufficient if the composite material is able to remain dispersed in the oil phase and the skin penetrability of the composite material is not impaired. For example, the average particle size may be from 1 nm or more to 1,000 nm or less, preferably from 10 nm or more to 900 nm or less, more preferably from 50 nm or more to 700 nm or less, and even preferably from 100 nm or more to 500 nm or less.

### (Leuprorelin)

Leuprorelin is typically used in a form of acetate salt, and is distributed in injectable form under the treadename of "Leuplin^{®}." The leuprorelin acetate is a peptide-based compound represented by the following formula (I):

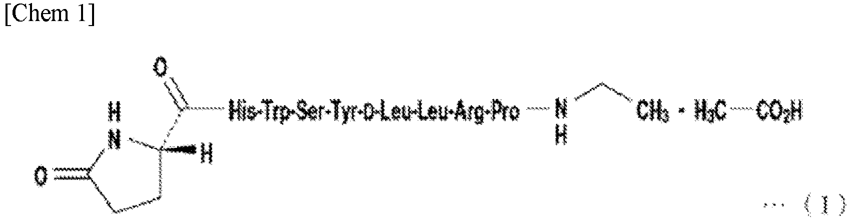

Leuprorelin shows the following properties: white to yellowish white powder, freely soluble in water or acetic acid, soluble in methanol, and slightly insoluble in ethanol. Leuprorelin is hygroscopic. Molecular weight of leuprorelin is 1209.42 (1269.45 for leuprorelin acetate). Due to such a high molecular weight over 1,000, besides being hydrophilic, this compound typically cannot penetrate through the skin by itself. Nevertheless, the transdermal absorption agent according to present embodiment has achieved an improvement of skin penetrability by incorporating leuprorelin into the S/O type particle.

The type of the salt of leuprorelin contained in the transdermal absorption agent is not particularly limited if it is a pharmaceutically acceptable salt thereof. The salt of leuprorelin is preferably an acid addition salt, which may be an inorganic acid salt or may be an organic acid salt. Examples of the inorganic acid salt include salts of inorganic acids including hydrohalic acids such as hydrochloric and hydrobromic acid, perchloric acid, nitric acid, and sulfuric acid. Examples of the organic acid salt include salts of organic acids including formic acid, acetic acid, propionic acid, lactic acid, pyroracemic acid, oxalic acid, malonic acid, and succinic acid. Among these salts, the salt of leuprorelin contained in the transdermal absorption agent is preferably acetic acid salt of leuprorelin (also called as leuprorelin acetate).

### (Surfactant)

Any surfactant described concerning Embodiment 1 may be applicable as the surfactant. The mass ratio of leuprorelin to the surfactant in the composite material is preferably from 1/100 or more to 1/10 or less, more preferably from 1/50 or more to 1/10 or less, even preferably from 1/50 or more to 1/20 or less, especially preferably from 1/50 or more to 1/30 or less. When the mass ratio of leuprorelin relative to the surfactant is not less than the above-described lower limit, the particle size can more effectively be controlled so as not to become too large, whereas when the mass ratio is not more than the above-described upper limit, the composite material can more effectively permeate into the skin.

The content of the composite material in the transdermal absorption agent is not particularly limited as long as the composite material remains dispersible. The content may be, for example, from 0.6 w/v% or more to 60 w/v% or less.

### (Stabilizing agent)

The composite material may contain a stabilizing agent. The stabilizing agent is preferably a hydrophilic protein or a polysaccharide having a molecular weight of 10,000 or greater. When the stabilizing agent is enclosed along with leuprorelin by the surfactant, the stability of the composite material may be improved and any incident such as leakage of leuprorelin outside of the composite material within the transdermal absorption agent may be avoided.

Examples of the protein as the stabilizing agent include serum albumin (molecular weight: approximately 67,000), ovoalbumin (molecular weight: approximately 45,000), casein (molecular weight: approximately 19,000 or greater), lysozyme (molecular weight: approximately 14,000 or greater), and lipase (molecular weight: approximately 45,000). Any of these proteins may be used alone or in combination of two or more. Among these proteins, serum albumin, ovoalbumin, or casein are preferred.

Examples of the polysaccharide include LM pectin, HM pectin, hydroxypropyl methylcellulose phthalate, heparin, alginic acid, and carboxymethyl cellulose. Any of these polysaccharides may be used alone or in combination of two or more. Among these polysaccharides, LM pectin, HM pectin, or hydroxypropyl methyl cellulose phthalate are preferred.

The molecular weight of the protein or the polysaccharide used as the stabilizing agent may vary depending upon their origin or other factors. For example, the molecular weight of the pectin is typically from 50,000 or greater to 150,000 or less, but pectins with molecular weights of from 20,000 or greater to 40,000 or less may be applicable. The molecular weight of the protein and the polysaccharide exemplified above may be said, in general, 10,000 or greater. The molecular weight of the protein and the polysaccharide used herein may be referred to manufacturer's catalogs. If the molecular weight is not known, it may be determined by a conventional technique and may be represented as a number average molecular weight or the like.

The mass ratio of the stabilizing agent to leuprorelin in the composite material is preferably from 0.01 or more to 100 or less, more preferably from 0.1 or more to 10 or less, and even preferably from 0.5 or more to 5 or less. When the mass ratio of the stabilizing agent to leuprorelin is not less than the above-described lower limit, the stabilizing agent may exert its effect more satisfactorily, whereas when the mass ratio is not more than the upper limit, the fraction of the composite material represented by leuprorelin may be sufficient, allowing the drug to exert its inherent effect more completely.

### (Oil phase)

Any oil phase described concerning Embodiment 1 may be used as the oil phase. The content of the oil phase in the transdermal absorption agent varies depending upon the specific oil component or other components, but is preferably from 50 w/v% or more to 99.5 w/v% or less, and more preferably from 60 w/v% or more to 90 w/v% or less.

### [Other component]

The transdermal absorption agent of the present embodiment may further contain the other components including the above-described transdermal absorption promoter.

### <Method for producing transdermal absorption agent>

The transdermal absorption agent may be produced, for example, by the method comprising the following steps (1) to (3):
(1) step of mixing leuprorelin with a solution containing a surfactant and, if necessary, a stabilizing agent in an organic solvent to prepare a W/O type emulsion (hereinafter sometimes referred to as "step (1)");
(2) step of drying the W/O type emulsion to prepare a composite material (hereinafter sometimes referred to as "step (2)"); and
(3) step of dispersing the composite material in an oil phase (hereinafter sometimes referred to as "step (3)").

### [Step (1)]

In step (1), first, an aqueous solution of leuprorelin is prepared. If necessary, stabilizing agent is also added thereto. The water used in this step is, for example, pure water, purified water, distilled water, saline solution, or buffer solution. If necessary, a small amount of a water-miscible organic solvent such as ethanol may be added thereto. Care must be taken so as not to add the solvent such as alcohol excessively because such solvents may make it difficult to form the emulsion. The concentrations of the leuprorelin and the stabilizing agent in the aqueous solution is not particularly limited as long as these can be dissolved in the solution substantially completely, but the concentration may be, for example, from about 0.1 mg/ml or more to about 30 mg/ml or less. Separately, a solution of the surfactant in an organic solvent is prepared. The organic solvent used in this step is not particularly limited as long as it can dissolve the surfactant and also can be removed by solvent diffusion in the subsequent step, but the examples include aliphatic hydrocarbons such as hexane and cyclohexane and aromatic hydrocarbons such as toluene. The concentration of the organic solvent is not particularly limited as well, but, for example, it may be from about 0.1 mg/ml or more to about 50 mg/ml or less.

Subsequently, a W/O type emulsion is prepared by mixing the aqueous solution of the leuprorelin and the stabilizing agent with the solution of the surfactant in the organic solvent, and then emulsifying and dispersing the resultant mixture in accordance with a conventional method. The emulsification and the dispersion may be performed by use of a conventional apparatus in the same manner as in Embodiment 1. The particle size of the dispersed droplets may also be controlled in the same manner as in Embodiment 1.

### [Step (2)]

In step (2), the W/O type emulsion obtained in step (1) is dried, to thereby obtain a composite material. The method for drying is not particularly limited. The examples of the drying method include lyophilization and vacuum drying, but lyophilization is preferred. The specific conditions for drying may follow those of the conventional method. Preferably, substantially all of the water and the organic solvent are removed in step (2). Water may cause leakage of leuprorelin within the preparation and organic solvents may be deleterious to the organism. Specifically, it is sufficient to reduce water content to around 1% by mass or less as measured by, for example, Karl Fischer titration.

### [Step (3)]

In step (3), the composite material obtained in the above step (2) as well as the other components such as the transdermal absorption promoter, if necessary, are dispersed in an oil phase, yielding the transdermal absorption agent according to the present embodiment as an S/O type solution or suspension. Specifically, as for the technique for dispersing, similar emulsification and dispersion techniques as used in Embodiment 1 may be employed. The amount of the oil phase used in step (3) depends upon factors such as compatibility between the surfactant and the oil phase, but, for example, from 4 ml or more to 200 ml or less per 1 gram of the composite material may be sufficient.

### <Dosage and administration>

The transdermal absorption agent according to the present embodiment may be applied directly to the affected area or other parts of the body, or alternatively, may be formulated with other additives. Examples of other additives include excipients (for example, sugars such as white sugar; starch derivatives such as dextrin; cellulose derivatives such as carmellose sodium; water-soluble polymers such as xanthan gum, and the like), colorants, lubricants (for example, metal salts of stearic acid such as calcium stearate and magnesium stearate; lauryl sulfates such as sodium lauryl sulfate and magnesium lauryl sulfate; starch derivatives mentioned above as the excipients, and the like), binders (for example, the above-described excipients, macrogol, and the like), emulsifiers, thickeners, wetting agents (for example, glycerin and the like), stabilizers (for example, para-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; acetic anhydride; sorbic acid; and the like), preservatives, solvents (for example, water, ethanol, glycerin, and the like), dissolution aids, suspending agents (for example, carmellose sodium and the like), buffering agents, pH regulating agents, and base materials (for example, polyethylene glycol, crotamiton, diethyl sebacate, petroleum jelly, and the like). These additives may be incorporated into the formulation in a conventional formulation amount.

The S/O type solution or suspension obtained by the above-described preparation method may be admixed with the other additives so as to obtain a preparation for external use by using a conventional technique in accordance with the intended dosage forms. The dosage forms of the preparation for external use include, but not limited to, ointments, lotions, aerosols, solid plasters, aqueous patches, creams, gels, plasters, reservoir-type skin patches, matrix-type skin patches, and tapes.

The dosage amount of the transdermal absorption agent according to the present embodiment may be adjusted depending upon factors such as the age and symptom of the patients, and the type of indication. For example, leuprorelin may be administered normally at least twice in an amount from about 1 µg or more to 5,000 µg or less, preferably from about 10 µg or more to 4,000 µg or less, more preferably from about 30 µg or more to 3,000 µg or less per dose per adult for a duration of from several weeks to several months.

### <Method for enhancing transdermal absorptivity of leuprorelin>

The method for enhancing transdermal absorptivity of leuprorelin according to one embodiment (hereinafter sometimes abbreviated as "the method of the present embodiment") includes enclosing leuprorelin with a surfactant to form a solid composite material, and then dispersing the composite material in an oil phase to form an external preparation. The specific conditions to implement the present embodiment may be referred to the description in the above "Transdermal absorption agent" section. The method of the present embodiment makes it possible to improve the transdermal absorption of leuprorelin, which has previously had difficulty in using in external preparations due to its low transdermal absorptivity which leads to an insufficient exertion of the drug efficacy.

In another embodiment, there is provided a transdermal absorption-type patch of Embodiment 1 in which the active ingredient is leuprorelin. The configuration of this transdermal absorption-type patch is identical to that of the transdermal absorption-type patch according to Embodiment 1 except that the active ingredient is leuprorelin. Therefore, as for the transdermal absorption-type patch containing leuprorelin, the description of the transdermal absorption-type patch according to Embodiment 1 may be referred to by replacing the term "active ingredient" with the term "leuprorelin."

### <Method of treatment>

The above-described transdermal absorption agent and the above-described transdermal absorption-type patch, each containing leuprorelin, may be used in the treatment of diseases for which leuprorelin is indicated. Examples of the diseases for which leuprorelin is indicated include prostate cancer, premenopausal breast cancer, estrogen-dependent diseases (such as endometriosis and uterine fibroids), and central precocious puberty.

In another embodiment, there is provided a method for treating prostate cancer comprising applying the above-described transdermal absorption agent or the above-described transdermal absorption-type patch, each containing a therapeutically effective amount of leuprorelin, to a patient or an affected animal in need of the treatment. Alternatively, in another embodiment, there is provided a method for treating premenopausal breast cancer comprising applying the above-described transdermal absorption agent or the above-described transdermal absorption-type patch, each containing a therapeutically effective amount of leuprorelin, to a patient or an affected animal in need of the treatment. Alternatively, in another embodiment, there is provided a method for treating estrogen-dependent diseases comprising applying the above-described transdermal absorption agent or the above-described transdermal absorption-type patch, each containing a therapeutically effective amount of leuprorelin, to a patient or an affected animal in need of the treatment.

Alternatively, in one embodiment, there is provided a method for treating central precocious puberty comprising applying the above-described transdermal absorption agent or the above-described transdermal absorption-type patch, each containing a therapeutically effective amount of leuprorelin, to a patient or an affected animal in need of the treatment. As used herein the term "therapeutically effective amount of leuprorelin" refers to the amount suggested above in connection with the above-described transdermal absorption agent as the dosage amount of leuprorelin, which amount depending upon the type of disease.

Alternatively, in one embodiment, there is provided the above-described transdermal absorption agent or the above-described transdermal absorption-type patch for treating prostate cancer. In one embodiment, there is provided the above-described transdermal absorption agent or the above-described transdermal absorption-type patch for treating premenopausal breast cancer. In one embodiment, there is provided the above-described transdermal absorption agent or the above-described transdermal absorption-type patch for treating estrogen-dependent diseases. In one embodiment, there is provided the above-described transdermal absorption agent or the above-described transdermal absorption-type patch for treating central precocious puberty.

The subject animals to be treated with the above-described transdermal absorption agent and the above-described transdermal absorption-type patch, each containing leuprorelin, are the same as those as in Embodiment 1, and are preferably vertebrates, and more preferably mammalian animals.

### Embodiment 3

In the following, the transdermal absorption agent according to Embodiment 3 is described. The transdermal absorption agent according to this embodiment contains octreotide or a salt thereof (hereinafter collectively referred to as "octreotide") as the active ingredient.

Octreotide is a therapeutic drug for diseases such as gastrointestinal hormone producing tumor and acromegaly. Currently, only injectable preparations are available for administering octreotide in clinical settings. Subcutaneous injection of octreotide requires administration multiple times a day. Administration by injection is painful, especially when administered multiple times a day, causing undue stress to the patient.

In order to reduce the number of injections, microsphere-type preparations have been developed that can confer sustained release properties to the preparation containing octreotide. Unexamined Japanese Patent Application Publication (Translation of PCT Application) No. 2017-509661 discloses a process for preparing a poly (D, L-lactide-co-glycolide) polymer microsphere of octreotide acetate. The microsphere-type preparation of octreotide is used in clinical settings as Sandostatin LAR^{®}.

The microsphere-type sustained release preparation of octreotide is administered once a month by intramuscular injection. However, the drug concentration does not reach a sufficient level for two weeks after the initial dose. For this reason, for the treatment of gastrointestinal hormone producing tumor, it is necessary to administer injectable solutions containing the same dose of octreotide concurrently. Therefore, patients are still subjected to heavy stress, and there remains a need for developing an octreotide preparation that improves the quality of life of patients.

One conceivable way to avoid administration by injection is to employ external preparations. In Unexamined Japanese Patent Application Publication (Translation of PCT Application) No. 2019-516709, administration of octreotide via transdermal route is suggested. However, the skin penetrability of octreotide is extremely low because octreotide is a hydrophilic compound. Therefore, even if octreotide is formulated into a transdermal absorption agent, its drug efficacy may not be sufficiently exerted.

As for octreotide, preparations that improve skin penetrability of octreotide as well as confer an excellent sustained release properties on octreotide have not previously been investigated.

The transdermal absorption agent according to the present embodiment is able to improve skin penetrability of octreotide as well as to confer an excellent sustained release properties on octreotide.

### <Transdermal absorption agent>

The configuration of the transdermal absorption agent according to the present embodiment is identical to that of the transdermal absorption agent according to Embodiment 2 except that the active ingredient is octreotide instead of leuprorelin. Therefore, concerning the transdermal absorption agent containing octreotide, the description of the transdermal absorption agent according to Embodiment 2 may be referred to by replacing the term "leuprorelin" with the term "octreotide." Transdermal absorption agent according to the present embodiment is described below mainly about the differences from the transdermal absorption agent according to Embodiment 2.

The transdermal absorption agent according to the present embodiment includes a solid composite material and an oil phase, in which the solid composite material is octreotide or a salt thereof (hereinafter collectively referred to as "octreotide") enclosed with a surfactant. The composite material is formed such that octreotide is associated with the hydrophilic portion of the surfactant whereby the octreotide is enclosed by the surfactant. In the transdermal absorption agent, the composite material is dispersed in the oil phase, forming an S/O type particle.

The octreotide is a peptide-based compound represented by the following formula (I). Octreotide is typically used in a form of acetate salt.

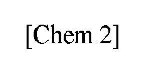

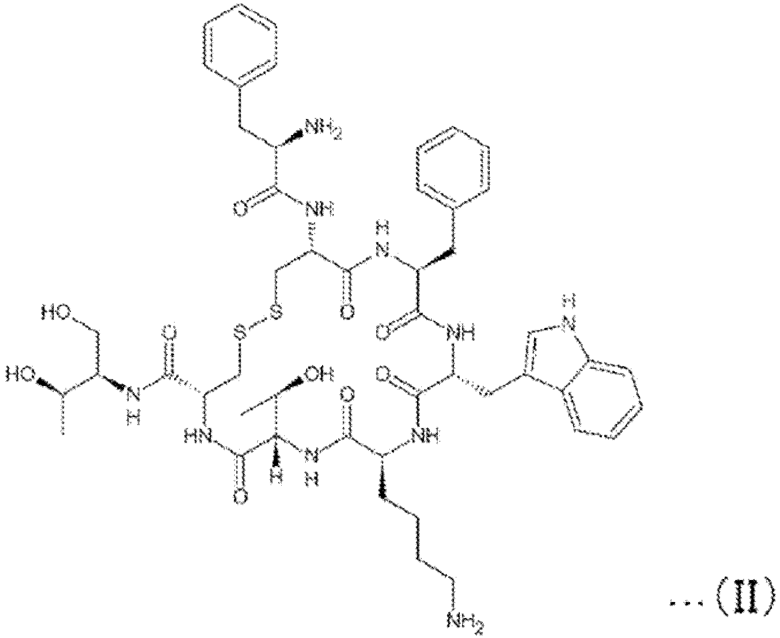

Octreotide acetate shows the following properties: white to slightly yellowish white powder with a slight acetic acid odor. Extremely soluble in water, and freely soluble in ethanol, acetic acid, or N-methylpyrrolidone. The molecular weights of octreotide and octreotide acetate are 1019.24 and 1139.34, respectively, that is, over 1,000. In addition, they are hydrophilic compounds. Therefore, they typically cannot penetrate through the skin by themselves. The skin penetrability of the octreotide in the transdermal absorption agent according to the present embodiment is improved through forming the octreotide into the S/O type particle.

The salt of octreotide is not particularly limited as long as it is pharmaceutically acceptable salt. The salts are preferably acid addition salts that may be inorganic salts or may be organic salts. Examples of the inorganic acid salt include salts of inorganic acids including hydrohalic acids such as hydrochloric and hydrobromic acid, perchloric acid, nitric acid, and sulfuric acid. Examples of the organic acid salt include salts of organic acids including formic acid, acetic acid, propionic acid, lactic acid, pyroracemic acid, oxalic acid, malonic acid, and succinic acid. The octreotide contained in the transdermal absorption agent according to the present embodiment is preferably octreotide acetate.

The mass ratio of the octreotide to the surfactant (parts by mass of the octreotide relative to 1 part by mass of the surfactant) in the composite material is preferably 0.01 to 0.2, more preferably 0.012 to 0.1, even preferably 0.015 to 0.05, and especially preferably 0.017 to 0.03. Suitably, the mass ratio of the octreotide to the surfactant is 0.018 to 0.025 or 0.02.

The configuration of the method for producing the transdermal absorption agent according to the present embodiment is also identical to that of the transdermal absorption agent according to Embodiment 2 except that the active ingredient is octreotide instead of leuprorelin.

The concentration of octreotide in the aqueous solution of octreotide in step (1) is not particularly limited as long as octreotide can be dissolved substantially completely, but the concentration is, for example, from 0.1 mg/ml or more to 30 mg/ml or less. The concentration of the surfactant in the solution of the organic solvent is not particularly limited, but the concentration is, for example, from 1 mg/ml to 100 mg/ml, from 20 mg/ml to 90 mg/ml, or from 40 mg/ml to 80 mg/ml.

The dosage of the transdermal absorption agent according to the present embodiment may be adjusted depending upon factors such as the age and symptom of the patients, and the type of indication. For example, octreotide may be administered normally at least twice in an amount from 1 µg or more to 30 mg or less, preferably from 10 µg or more to 10 mg or less, more preferably from 30 µg or more to 3 mg or less per dose per adult for a duration of from several weeks to several months.

In the transdermal absorption agent according to the present embodiment, a solid octreotide-surfactant composite material is dispersed in an oil phase, forming an S/O type particle. As a result of that, the transdermal absorption agent is enabled to allow octreotide, which would hardly penetrate through the skin, to permeate through the skin into the body as shown in Example below. Furthermore, the transdermal absorption agent has better sustained release properties than conventional injectable forms as shown in Example below.

In addition, owing to its pharmacokinetic properties exhibiting sustained release properties, the transdermal absorption agent according to the present embodiment can suppress transient hypoglycemia, which has been reported as a side effect of octreotide, as shown in Example below.

In another embodiment, there is provided a method for enhancing transdermal absorptivity of octreotide. The method includes enclosing octreotide with a surfactant to form a solid octreotide-surfactant composite material, and dispersing the composite material in an oil phase to form an external preparation. The method of the present embodiment enables to improve transdermal absorptivity of octreotide, which has previously had difficulty in using in an external preparation due to its low transdermal absorptivity which leads to an insufficient exertion of the drug efficacy.

### <Method for enhancing transdermal absorptivity of octreotide>

The method for enhancing transdermal absorptivity of octreotide according to one embodiment includes enclosing octreotide with a surfactant to form a solid composite material, and dispersing the composite material in an oil phase to form an external preparation.

In another embodiment, there is provided a transdermal absorption-type patch of Embodiment 1 in which the active ingredient is octreotide. The configuration of this transdermal absorption-type patch is identical to that of the transdermal absorption-type patch according to Embodiment 1 except that the active ingredient is octreotide. Therefore, concerning the transdermal absorption-type patch containing octreotide, the description of the transdermal absorption-type patch according to Embodiment 1 may be referred to by replacing the term "active ingredient" with the term "octreotide."

### <Method of treatment>

The above-described transdermal absorption agent and the above-described transdermal absorption-type patch, each containing octreotide, may be used in the treatment of diseases for which octreotide is indicated. Diseases for which octreotide is indicated include, for example, VIP-secreting tumor, carcinoid tumor, gastrin-producing tumor, gastrointestinal hormone producing tumor, acromegaly, pituitary gigantism, gastrointestinal neuroendocrine tumor, gastrointestinal symptoms associated with gastrointestinal obstruction in palliative care for advanced and recurrent cancer patient, thyroid stimulating hormone secreting pituitary adenoma, diarrhea and flushing caused by carcinoid syndrome, and diarrhea caused by vasoactive intestinal peptide tumors.

In another embodiment, there is provided a method for treating the above-described diseases including VIP-secreting tumor comprising applying the above-described transdermal absorption agent or the above-described transdermal absorption-type patch, each containing a therapeutically effective amount of octreotide, to a subject in need of the treatment. As used herein the term "therapeutically effective amount of octreotide" refers to the amount suggested above in connection with the above-described transdermal absorption agent as the dosage amount of octreotide, which amount depending upon the type of disease.

In another embodiment, there is provided the above-described transdermal absorption agent or the above-described transdermal absorption-type patch for treating diseases such as VIP secreting tumor.

### Examples

The present disclosure is described below with reference to Examples, but the present disclosure is not limited thereto.

### [Example 1]

### (Preparation of self-adhesive sheet with release liner containing FITC-OVA-encapsulated S/O type preparation)

### 1. Preparation of S/O type preparation

First, an S/O type preparation was prepared. Specifically, a solution (0.5 mg/ml) was prepared by dissolving FITC-labeled ovalbumin (OVA; weight average molecular weight: approximately 45,000) (hereinafter sometimes abbreviated as "FITC-OVA") in distilled water. The resultant solution was mixed with a cyclohexane solution containing a cyclohexane solution containing sucrose laurate L-195 (RYOTO SUGAR ESTER, manufactured by Mitsubishi Chemical Foods Corporation) in a volume ratio of 1:2. Subsequently, the resultant mixture was stirred by use of Polytron homogenizer (manufactured by KINEMATICA AG) at 26,000 rpm for 2 minutes to prepare a W/O type emulsion. The resultant W/O type emulsion was lyophilized, to thereby obtain a solid L195-FITC-OVA composite material. Subsequently, to the L195-FITC-OVA composite material was added isopropyl myristate (manufactured by FUJIFILM Wako Pure Chemical Co., Ltd.), to thereby obtain the FITC-OVA-encapsulated S/O type preparation (solid content: 30% by mass).

### 2. Preparation of self-adhesive sheet with release liner containing S/O type preparation

The FITC-OVA-encapsulated S/O type preparation obtained in "1." was mixed with DURO-TAK 87-4098 (acrylic copolymer, solid content: 38.5% by mass, manufactured by Henkel AG & Co. KGaA) as an adhesive component in a mass ratio of 1:1. Subsequently, the resultant mixture was mixed thoroughly with a vortex mixer (VORTEX-GENIE 2 Mixer, manufactured by M&S Instruments Inc.) until a homogeneous mixture was obtained, yielding an adhesive solution containing the S/O type preparation. The obtained adhesive solution containing the S/O type preparation was applied to a liner layer (liner OPP 40 µm), and was dried at 60°C for about 30 minutes to form an adhesive layer on the liner layer. Subsequently, a support material (sheet made of acrylic resin, thickness: 50 µm) was attached to the surface of the adhesive layer opposite to the surface in contact with the liner layer, to thereby obtain a self-adhesive sheet with a release liner containing the S/O type preparation (hereinafter sometimes abbreviated as "FITC-OVA-encapsulated S/O self-adhesive sheet"; FITC-OVA content per sheet: 14.2 µg).

### [Test Example 1]

### (Test of S/O self-adhesive sheet for permeability into pig skin)

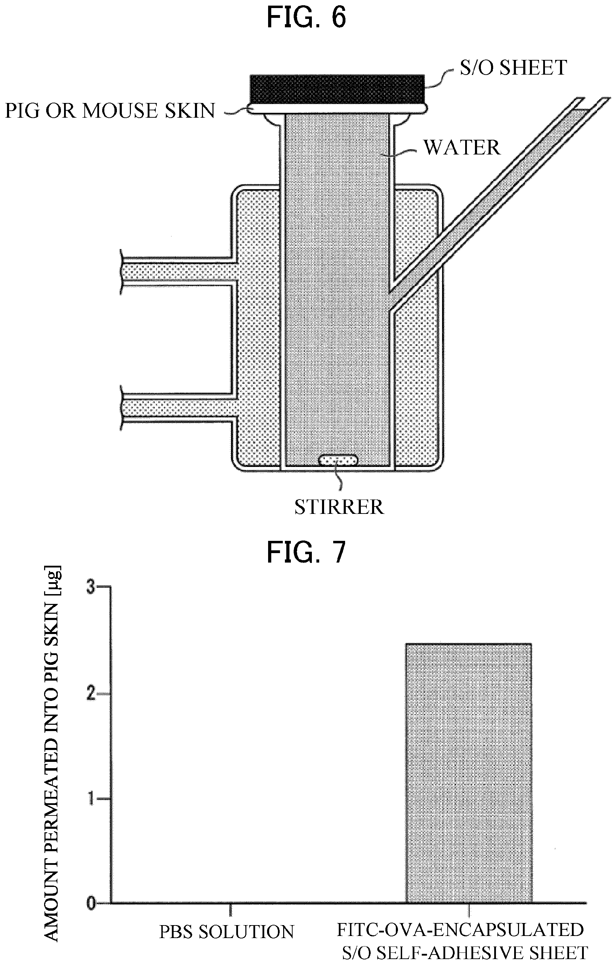

A test of FITC-OVA-encapsulated S/O self-adhesive sheet for permeability into pig skin was performed by using the transdermal absorption testing system shown in FIG. 6. Specifically, a jacketed static Franz diffusion cell for membrane permeability assay (manufactured by CosMED Pharmaceutical) was filled with water, and a piece of pig skin was placed over an opening of the top surface of the diffusion cell. Then, the FITC-OVA-encapsulated S/O self-adhesive sheet was overlaid on the pig skin so that the adhesive layer was attached to the pig skin, followed by allowing to stand at room temperature for about 24 hours. The surface of the resultant pig skin was rinsed several times with ethanol and phosphate buffered saline (PBS), and then the skin was shredded and immersed in 500 µl of extraction solution (PBS: acetonitrile: methanol = 2:1:1 by volume). The resultant mixture was shaken for 24 hours, and the resultant extraction solution was filtered through a 0.2 µm filter. The concentration of FITC-OVA in the filtrate was quantified by using fluorescence spectrophotometer (LS-55, manufactured by PerkinElmer, Inc.) and the amount of the FITC-OVA permeated into the pig skin was calculated from the result. As a control, a PBS solution containing 14.2 µg of FITC-OVA, the same amount as that contained in the FITC-OVA-encapsulated S/O self-adhesive sheet, was applied to another piece of pig skin and the test of permeability was performed. The results are shown in FIG. 7. The permeation rate ([amount of FITC-OVA permeated into the skin]/[total amount of FITC-OVA originally contained in the self-adhesive sheet] × 100) was found to be 17.2 %.

As shown in FIG. 7, it was confirmed that the amount permeated into the pig skin from the FITC-OVA-encapsulated S/O self-adhesive sheet was higher than that of the PBS solution.

### [Examples 2 and 3]

### (Preparation of self-adhesive sheet with release liner containing antibody- or peptide-encapsulated S/O type preparation)

The procedure in Example 1 was repeated except that FITC-labeled goat anti-mouse IgG (ab 97022, manufactured by Abcam, weight average molecular weight: approximately 150,000) (hereinafter sometimes abbreviated as "FITC-IgG") (0.5 mg/ml) or octreotide (OCT, manufactured by FUJIFILM Wako Pure Chemicals, weight average molecular weight: approximately 1,080) (0.5 mg/ml) was used, to thereby obtain a self-adhesive sheet of FITC-IgG-encapsulated S/O type preparation with a release liner (hereinafter sometimes abbreviated as "FITC-IgG-encapsulated S/O self-adhesive sheet"; FITC-IgG content per sheet: 43.9 µg) or a self-adhesive sheet of OCT-encapsulated S/O type preparation with a release liner (hereinafter sometimes abbreviated as "OCT-encapsulated S/O self-adhesive sheet"; OCT content per sheet: 100 µg).

### [Test Example 2]

### (Test of S/O self-adhesive sheet for permeability into pig skin and penetrability through mouse skin)

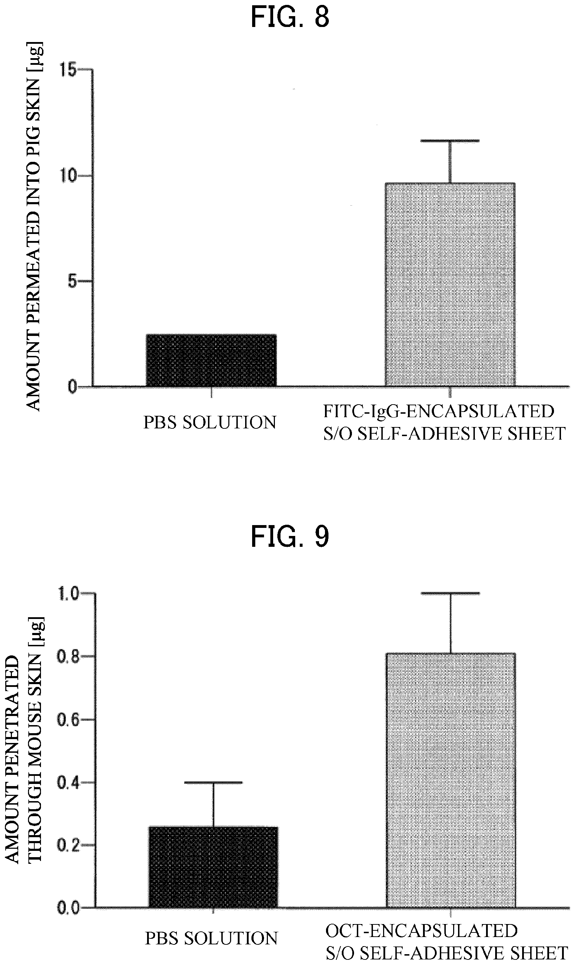

A test for permeability into pig skin was performed in the same manner as described in Test Example 1 by using the self-adhesive sheet of FITC-IgG-encapsulated S/O type preparation obtained in Example 2. As a control, a PBS solution containing 43.9 µg of FITC-IgG, the same amount as that contained in the FITC-IgG-encapsulated S/O self-adhesive sheet, was applied to another piece of pig skin and the test of permeability into the pig skin was performed. The results are shown in FIG. 8. The permeation rate ([amount of FITC-IgG permeated into the skin]/[total amount of FITC-IgG originally contained in the self-adhesive sheet] × 100) was found to be 21.7 %.

Additionally, test of penetrability through mouse skin was also performed on the self-adhesive sheet of OCT-encapsulated S/O type preparation obtained in Example 3 by using the transdermal absorption testing system. Specifically, a jacketed static Franz diffusion cell for membrane permeability assay (manufactured by CosMED Pharmaceutical) was filled with water, and a piece of mouse skin was placed over an opening of the top surface of the diffusion cell. Then, the OCT-encapsulated S/O self-adhesive sheet was overlaid on the mouse skin so that the adhesive layer was attached to the mouse skin, followed by allowing to stand at room temperature for about 24 hours. Subsequently, a portion of the solution was taken from the stationary Franz diffusion cell, and the amount of OCT contained in the solution was quantified by liquid chromatography (HPLC, manufactured by Shimadzu Corporation). The amount penetrated through the mouse skin was calculated from the results. As a control, a PBS solution containing 100 µg of OCT, the same amount as that contained in the OCT-encapsulated S/O self-adhesive sheet, was applied to another piece of mouse skin and the test of penetrability through the mouse skin was performed. The results are shown in FIG. 9. The penetration rate ([amount of OCT penetrated through the skin]/[total amount of OCT originally contained in the self-adhesive sheet] × 100) was found to be 0.8%.

As shown in FIG. 8, it was confirmed that the amount permeated into the pig skin from the FITC-IgG-encapsulated S/O self-adhesive sheet was higher than that of the PBS solution. As shown in FIG. 9, it was also confirmed that the amount penetrated through the mouse skin from the OCT-encapsulated S/O self-adhesive sheet was higher than that of the PBS solution.

### [Test Example 3]

### (Animal immunization test using S/O self-adhesive sheet)

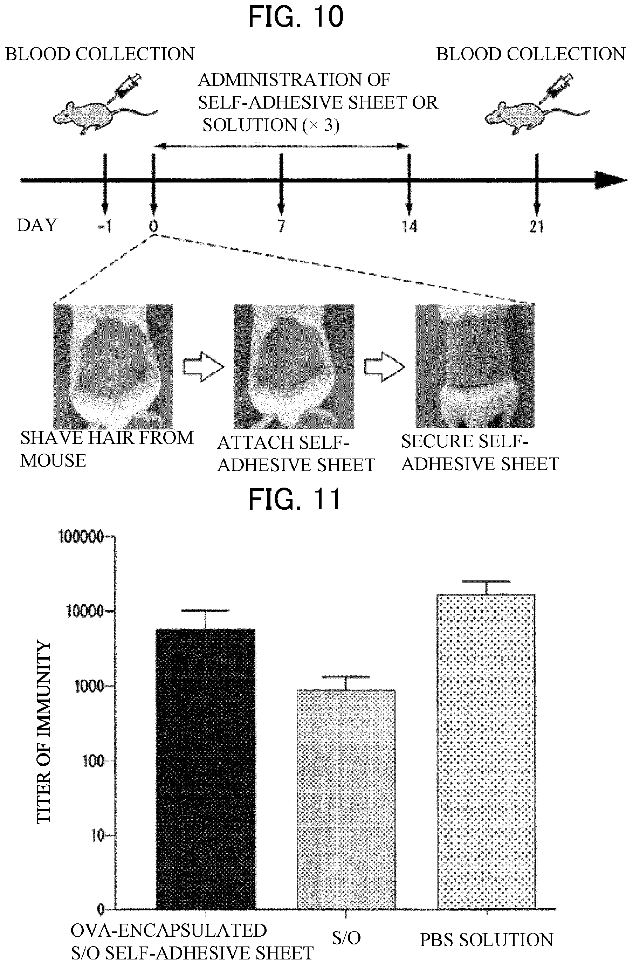

Animal immunization test was performed in accordance with the time schedule shown in FIG. 10. Specifically, first, hair on the back of mouse was shaved with a clipper three days before the immunization test started. Subsequently, the blood was collected via the caudal vein on the day before the immunization test started. Subsequently, on the following day, the OVA-encapsulated S/O self-adhesive sheet obtained in Example 1 was attached to the shaved back skin. The OVA-encapsulated S/O self-adhesive sheet was secured with kinesiology tape (manufactured by Nichiban Corporation). The OVA-encapsulated S/O self-adhesive sheet was replaced with new ones on day 7 and day 14 from the start of the immunization test, administered three times in total. On the 21st day from the start of the immunization test, blood samples were collected via the caudal vein. As a control 1, a PBS solution containing 30 µg of OVA, the same amount as that contained in the S/O self-adhesive sheet, was injected subcutaneously. Administrations were performed at the same time points as the time schedule for administration of the OVA-encapsulated S/O self-adhesive sheet, administered three times in total. In addition, as a control 2, the OVA-encapsulated S/O type preparation (in a form of liquid) prepared in Example " 1." was applied to the shaved back skin of mouse so that 30 µg of OVA, the same amount as that contained in the OVA-encapsulated S/O self-adhesive sheet, was applied. Administrations were performed at the same time points as the time schedule for administration of the OVA-encapsulated S/O self-adhesive sheet, administered three times in total. The amount of anti-OVA antibody in the blood was examined by ELISA. Specifically, the test was performed in accordance with the following procedure.

First, to a 96-well microplate (maxisorp nunc-immuno plate, manufactured by Thermo Fisher Scientific, Inc.) was added an aqueous solution containing 5.0 mg/ml of OVA in Milli Q water at 100 µl/well, and the plate was incubated at 4°C overnight. Subsequently, in order to avoid non-specific adsorption to the plate, a PBS solution containing 2% by mass of BSA was added to each well at 200 µl/well for blocking, followed by incubation at 37°C for 2 hours. Serum was diluted with a PBS solution containing 2% by mass of BSA at an appropriate dilution ratio, and the resultant solution was added to the plate at 20 µl/well, followed by incubation at 37°C for 2 hours. Subsequently, a solution (1 mg/ml) of horseradish peroxidase (HRP)-conjugated secondary antibody (Anti-Mouse IgG-HRP, manufactured by Rockland Immunochemicals, Inc.) was 20,000-fold diluted with a PBS solution containing 2% by mass of BSA, and the resultant solution was added to the plate at 100 µl/well, followed by incubation at 37°C for 2 hours. Subsequently, 3,3',5,5'-tetramethylbenzidine (TMB, manufactured by Thermo Fisher Scientific, Inc.) was added to each well at 100 µl/well, followed by incubation at 37°C for 30 min. Subsequently, 1MHCl was added to each well at 50 µl/well. The O.D. value at 450 nm was read, and the antibody titer was calculated therefrom. The results are shown in FIG. 11.

As shown in FIG. 11, it has been demonstrated that the amount of anti-OVA antibody induced by the OVA-encapsulated S/O self-adhesive sheet was higher than that of the mouse group to which the OVA-encapsulated S/O type preparation (in a form of liquid) was applied, and was equivalent to that of the mouse group to which the PBS solution containing 30 µg of OVA, the same amount as that contained in the OVA-encapsulated S/O self-adhesive sheet, was subcutaneously injected.

### [Test Example 4]

### (Animal immunization test using S/O self-adhesive sheet)

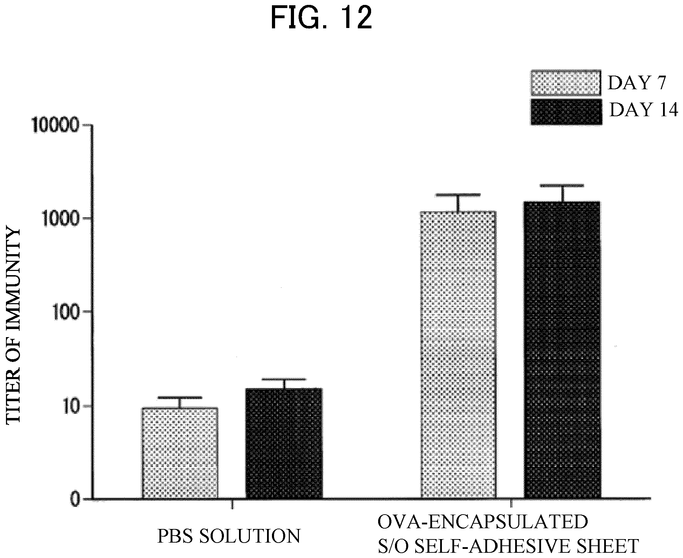

The procedure described in Example 1 was repeated except that 0.5 mg/ml of OVA was used, to thereby obtain a self-adhesive sheet of OVA-encapsulated S/O type preparation with a release liner (hereinafter sometimes abbreviated as "OVA-encapsulated S/O self-adhesive sheet"; OVA content per sheet: 20 µg). The immunization test was performed in the same manner as described in Example 3 except that the thus-obtained OVA-encapsulated S/O self-adhesive sheet (OVA content per sheet: 20 µg) was used. As a control, a PBS solution containing 20 µg of OVA, the same amount as that contained in the OVA-encapsulated S/O self-adhesive sheet, was applied to the shaved back skin of the mouse. Blood samples were collected on day 7 and day 14 after the last administration, and the amount of anti-OVA antibody in the blood samples were examined in the same manner as in Test example 3. The results are shown in FIG. 12.

As shown in FIG. 12, it has been demonstrated that the amount of anti-OVA antibody induced by the OVA-encapsulated S/O self-adhesive sheet was higher than that of the mouse group to which the PBS solution was applied.

### [Example 4]

### (Preparation of S/O type preparation)

To a leuprorelin acetate solution containing 1 mg of leuprorelin acetate dissolved in 2 ml of distilled water was added 4 ml of a cyclohexane solution containing sucrose laurate (L-195, lauric acid: 99% by mass, HLB: 1, manufactured by Mitsubishi Chemical Foods Co., Ltd.) in a concentration of 12.5 mg/ml or 2.5 mg/ml so that the mass ratio of leuprorelin acetate to the surfactant was 1/50 or 1/10, and the resultant mixture was agitated in a high speed at 26,000 rpm for 2 minutes with a homogenizer, to thereby obtain a W/O type emulsion. The emulsion was lyophilized for an entire day, to thereby obtain a leuprorelin acetate-surfactant composite material.

To this composite material was added 1 ml of isopropyl myristate (manufactured by Tokyo Kasei Kogyo Co., Ltd.) so as to disperse the composite material therein, to thereby obtain S/O type preparations having two different contents of the surfactant. Both of the S/O type preparations contained leuprorelin acetate in a concentration of 1 mg/ml.

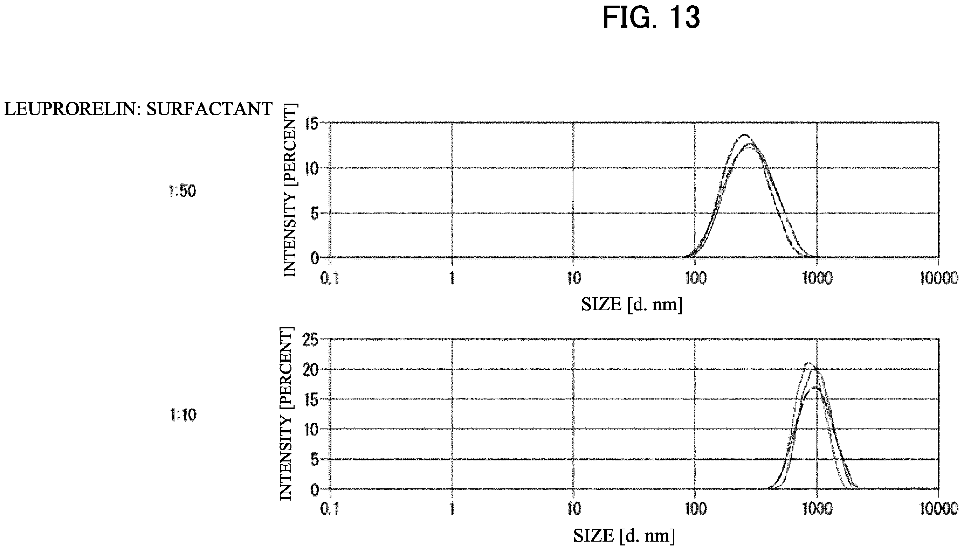

FIG. 13 shows the particle size distribution (frequency distribution) of the leuprorelin acetate-surfactant composite material in each S/O type preparation obtained by the method described above. Table 1 shows the average particle size and the polydispersity index (PDI) of the leuprorelin acetate-surfactant composite material in each S/O type preparation. The particle size distribution and the average particle size were determined by laser scattering technique (using Nano ZS instrument manufactured by Sysmex Corporation).

**[Table 1]**

| Leuprorelin: Surfactant | Average Particle Size [nm] | PDI |
|---|---|---|
| 1:50 | 231 | 0.022 - 0.11 |
| 1:10 | 880 | 0.068 - 0.167 |

As shown in FIG. 13 and Table 1, two different S/O type preparations containing the leuprorelin acetate-surfactant composite material dispersed therein having a uniform particle size with an average particle size ranging from 231 nm or greater to 880 nm or less were obtained.

### [Example 5]

### (Preparation of S/O type preparation)

To a leuprorelin acetate solution containing 1 mg of leuprorelin acetate dissolved in 2 ml of distilled water was added 4 ml of a cyclohexane solution containing sucrose laurate (L-195, lauric acid: 99% by mass, HLB: 1, manufactured by Mitsubishi Chemical Foods Co., Ltd.) in a concentration of 12.5 mg/ml so that the mass ratio of leuprorelin acetate to the surfactant was 1/50, and the resultant mixture was agitated in a high speed at 26,000 rpm for 2 minutes with a homogenizer, to thereby obtain a W/O type emulsion. The emulsion was lyophilized for an entire day, to thereby obtain a leuprorelin acetate-surfactant composite material.

To this composite material were added 0.9 ml of isopropyl myristate (manufactured by Tokyo Kasei Kogyo Co., Ltd.) and 0.1 ml of glyceryl monooleate (MGO) so as to disperse the composite material therein, to thereby obtain an S/O type preparation. The thus-obtained S/O type preparation contained leuprorelin acetate in a concentration of 1 mg/ml.

### [Test Example 5]

### (Test of penetrability for S/O type preparation through mouse skin)

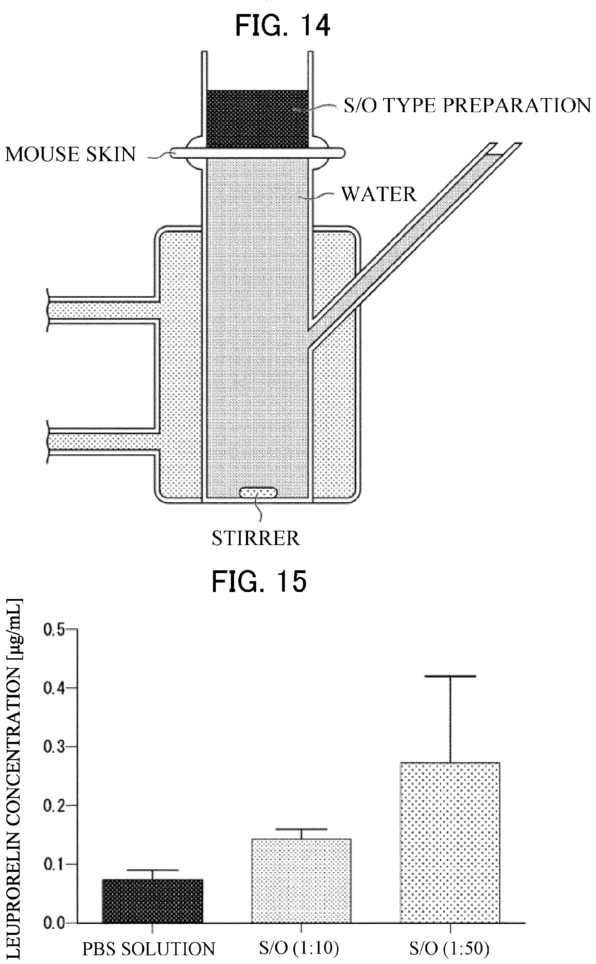

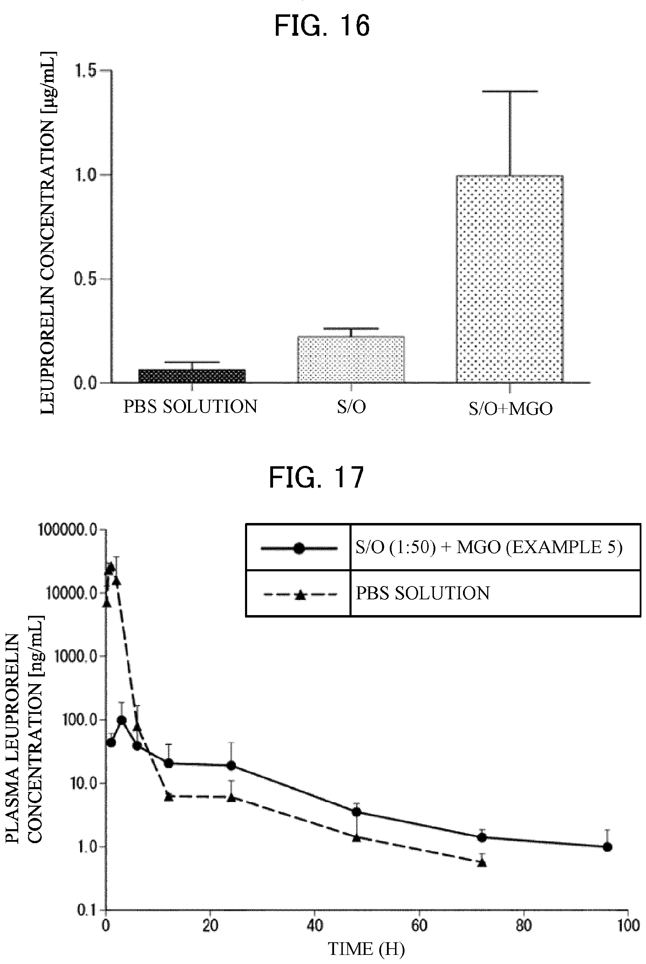

Tests of penetrability for the S/O type preparations obtained in Examples 4 and 5 through mouse skin were performed by using the transdermal absorption testing system shown in FIG. 14. Specifically, a jacketed static Franz diffusion cell for membrane permeability assay (manufactured by CosMED Pharmaceutical) was filled with water, and a piece of mouse skin was placed over an opening of the top surface of the diffusion cell. Then, 0.2 ml of the S/O type preparation was placed on the mouse skin, followed by allowing to stand at room temperature for about 24 hours. Subsequently, a portion of the solution was taken from the stationary Franz diffusion cell, and the concentration of leuprorelin (LEU) in the solution was quantified by liquid chromatography-mass spectrometer (LC-MS, Shimadzu Corporation). As a control, 0.2 ml of a PBS solution was placed on the mouse skin, and the test of penetrability through the mouse skin was performed in the same manner as described above. FIG. 15 shows the test results for two S/O type preparations having two different contents of the surfactant, and FIG. 16 shows the test results for two different S/O type preparations with and without MGO.

From the results shown in FIG. 15, the penetrability of the S/O type preparations through the mouse skin was confirmed. The penetrability of leuprorelin through the mouse skin was especially high when the mass ratio of leuprorelin acetate to the surfactant in the S/O type preparation was 1/50. From the results in FIG. 16, it was clearly shown that the amount of leuprorelin penetrated through the mouse skin was increased by about 5 times through the incorporation of MGO, indicating that the penetrability through the mouse skin was promoted by incorporating MGO into the S/O type preparation.

### [Test Example 6]

### (Pharmacokinetic study of leuprorelin)

Hair on the back of mouse was shaved with a clipper three days before the study started. Subsequently, the S/O type preparation obtained in Example 5 was applied to a patch (1.5 cm × 3 cm) so that leuprorelin acetate was administered in an amount of 2 mg, and then the resultant patch was applied to the shaved back skin. Blood samples were collected via the ocular fundus of the mouse at 0.25, 0.5, 1, 2, 6, 12, 24, 48, 72, and 96 hours after the administration. As a control group, a PBS solution in which 2 mg of leuprorelin acetate was dissolved was subcutaneously injected. Plasma concentration of leuprorelin (in ng/ml) was quantified by LC-MS (manufactured by Shimadzu Corporation). The results are shown in FIG. 17. In addition, pharmacokinetic analysis was performed on the collected blood samples using Phoenix^{®} WinNonlin^{®} (manufactured by CERTARA, Inc.; complying with the requirements of the U.S. Food and Drug Administration (FDA)). The analytical results are shown in Table 2.

**[Table 2]**

| | Cmax [ng/ml] | Tₘₐₓ [h] | AUC [ng·h/ml] | AUC₆ₕ₋₉₆ₕ [ng·h/ml] | AUC₁₂ₕ₋₉₆ₕ [ng·h/ml] |
|---|---|---|---|---|---|
| Injection | 26632 | 1 | 71838 | 452.4 | 195.6 |
| S/O | 98.5 | 3 | 1146 | 776.4 | 597.6 |

As shown in FIG. 17 and Table 2, it was confirmed that the plasma concentration of leuprorelin of the mouse to which the S/O type preparation was administered was exceeded that of the mouse to which the injectable preparation was administered at 12 hours from the start of administration and later, indicating that the prolonged release properties of drug efficacy (sustainability) of the S/O type preparation was superior to that of the injectable preparation.

### [Example 6]

### (Preparation of S/O type preparation)

To an octreotide acetate solution containing 1 mg of octreotide acetate dissolved in 2 ml of distilled water was added 4 ml of a cyclohexane solution containing sucrose laurate (L-195, lauric acid: 99% by mass, HLB: 1, manufactured by Mitsubishi Chemical Foods Co., Ltd.) in a concentration of 12.5 mg/ml so that the mass ratio of octreotide acetate to the surfactant was 1/50, and the resultant mixture was agitated at 26,000 rpm for 2 minutes with a homogenizer. The resultant W/O type emulsion was lyophilized for an entire day, to thereby obtain an octreotide acetate-surfactant composite material.

To this composite material was added 1 ml of isopropyl myristate (manufactured by Tokyo Kasei Kogyo Co., Ltd.) so as to disperse the composite material therein, to thereby obtain an S/O type preparation. The S/O type preparation contained octreotide acetate in a concentration of 1 mg/ml. The particle size distribution (frequency distribution) and average particle size of the octreotide acetate-surfactant composite material in the S/O type preparation were determined by dynamic light scattering technique (Zetasizer Nano ZS, manufactured by Malvern Panalytical).

### (Results)

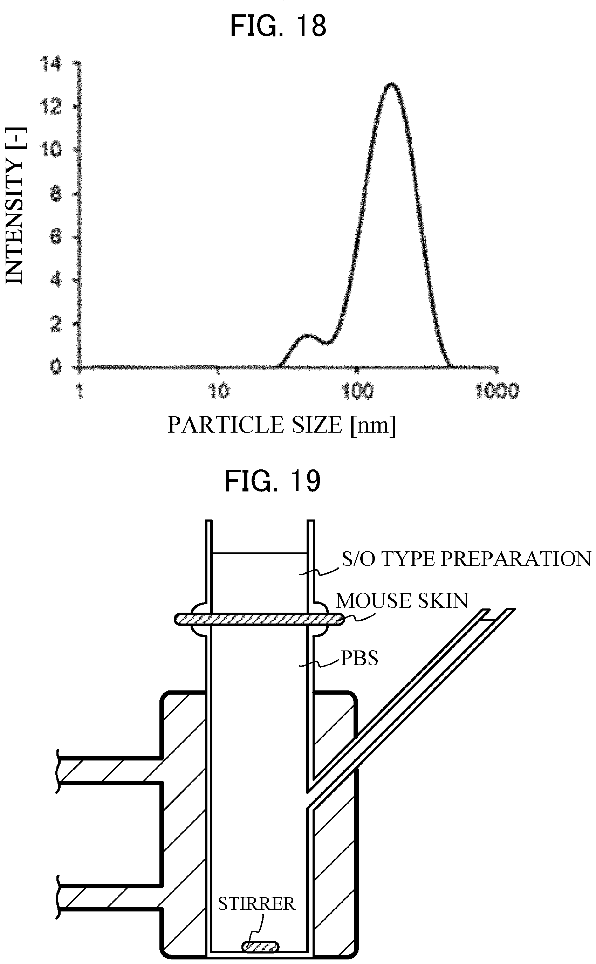

FIG. 18 shows the particle size distribution of the octreotide acetate-surfactant composite material in the S/O type preparation. The average particle size of the octreotide acetate-surfactant composite material in the S/O type preparation was found to be 145.0 nm with the polydispersity index (PDI) of 0.235 to 0.283.

It was shown that the S/O type preparation containing an octreotide acetate-surfactant composite material dispersed therein having an average particle size of 145.0 nm with a low polydispersity index was obtained in this Example.

### [Test Example 7]

### (Test of penetrability through mouse skin for S/O type preparation)

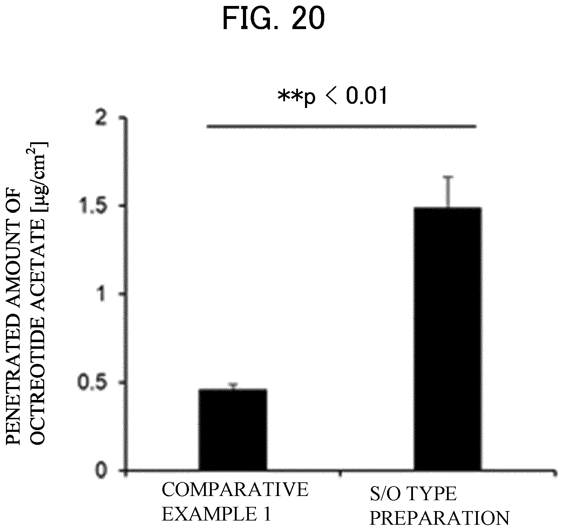

A test of penetrability of octreotide acetate through mouse skin (Laboskin, manufactured by Hoshino Laboratory Animals, Inc.) for the S/O type preparation obtained in Example 6 was performed by using the transdermal absorption testing system shown in FIG. 19. A jacketed static Franz diffusion cell for membrane permeability assay (manufactured by CosMED Pharmaceutical) was filled with phosphate buffered saline (PBS), and a piece of mouse skin was placed over an opening of the top surface of the diffusion cell, and then 0.2 ml of the S/O type preparation was placed on the mouse skin. After being allowed to stand at 32.5°C for 24 hours while stirring the PBS with a stirrer, a portion of the PBS was withdrawn from the receiver phase of the Franz diffusion cell, and the concentration of octreotide acetate in the withdrawn PBS was quantified by liquid chromatography-mass spectrometer (LC-MS, manufactured by Shimadzu Corporation). As a control, a predetermined amount of octreotide acetate was dissolved in phosphate buffered saline (PBS) so as to obtain an aqueous solution of octreotide acetate having a final concentration of 1 mg/ml (Comparative example 1). This solution was placed on the mouse skin in an amount of 0.2 ml and the test of the penetrability through the mouse skin was performed in the same manner as described above. FIG. 20 shows the amounts penetrated through the skin from the S/O type preparation and from Comparative Example 1 after 24 hours.

### (Results)

From the results shown in FIG. 20, it was confirmed that the amount of octreotide acetate penetrated from the S/O type preparation was significantly higher (p < 0.01) than that of Comparative example 1.

### [Example 7]

### (Preparation of S/O type preparation)

To an octreotide acetate solution containing 10 mg of octreotide acetate dissolved in 4 ml of distilled water was added 8 ml of a cyclohexane solution containing sucrose laurate (L-195, lauric acid: 99% by mass, HLB: 1, manufactured by Mitsubishi Chemical Foods Co., Ltd.) in a concentration of 62.5 mg/ml so that the mass ratio of octreotide acetate to the surfactant was 1/50, and then the octreotide acetate-surfactant composite material was prepared from the resultant mixture in the same manner as in Example 6.

To this composite material was added 1 ml of isopropyl myristate (manufactured by Tokyo Kasei Kogyo Co., Ltd.) so as to disperse the composite material therein, to thereby obtain an S/O type preparation. The S/O type preparation contained octreotide acetate in a concentration of 10 mg/ml.

### [Test Example 8]

### (Pharmacokinetic study of octreotide acetate)

Six-week-old mice (BALB/c, female) were acclimatized in an environment with free access to water and food for one week. Thereafter the backs of the mice were completely depilated by using a clipper and a depilatory cream three days before the study started. Then the mice were divided into the following three groups (3 mice per group) to which 2 mg of octreotide acetate was administered per mouse.

For S/O type preparation transdermal administration group, a patch was prepared by impregnating 200 µl of the S/O type preparation prepared in Example 7 into a three-layered gauze sheet (20 mm in length × 20 mm in width), and the resultant patch was attached to the back of each mouse and secured with a plastic tape, followed by removal after 24 hours.

For PBS solution injection group, 100 µl of an octreotide acetate solution in PBS (20 mg/ml) was subcutaneously injected to each mouse. For sustained release preparation injection group, 2 mg of Sandostatin LAR^{®} (manufactured by Novartis Pharma AG) was intramuscularly injected into the femoral region of the right hindlimb of each mouse.

Blood samples were collected via the ocular fundus of each mouse at 0.25, 0.5, 1, 2, 6, 12, 24, 48, and 96 hours after the administration. The plasma concentration of octreotide acetate was quantified by LC-MS (manufactured by Shimadzu Corporation).

Pharmacokinetic analysis of the time course of blood drug concentration on the blood samples was performed using Phoenix^{®} WinNonlin^{®} (manufactured by CERTARA, Inc.; complying with the requirements of the U.S. Food and Drug Administration (FDA)).

### (Results)

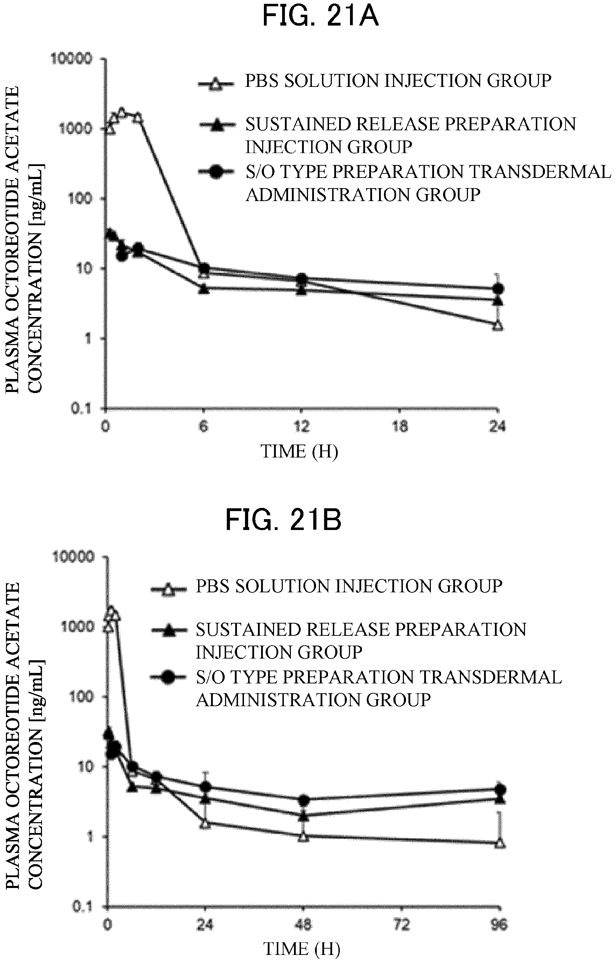

FIG. 21A and FIG. 21B show the time courses of the plasma concentration of octreotide acetate from 0 to 24 hours and from 0 to 96 hours, respectively. At 6 hours after the administration, the plasma concentration of octreotide acetate in mouse to which the S/O type preparation was transdermally administered exceeded that of the PBS solution injection group and thereafter stayed at higher plasma concentration level. The results of pharmacokinetic analysis are shown in Table 3.

**[Table 3]**

| | Cmax [ng/ml] | Tₘₐₓ [h] | AUC (0-96 h) [ng·h/ml] | AUC (6-96 h) [ng·h/ml] |
|---|---|---|---|---|
| S/O type preparation transdermal administration group | 19.3 | 2 | 509.0 | 424.7 |
| PBS solution injection group | 1726.5 | 1 | 5967.9 | 170.3 |
| Sustained release preparation injection group | 32.1 | 0.25 | 368.6 | 279.7 |

It has been demonstrated that the S/O type preparation had similar pharmacokinetics to that of Sandostatin LAR^{®}, a sustained release preparation, and had a superior prolonged release properties of drug efficacy (sustainability).

### [Example 8]

### (Preparation of S/O type skin patch)

To an octreotide acetate solution containing 10 mg of octreotide acetate dissolved in 4 ml of distilled water was added 8 ml of a cyclohexane solution containing sucrose laurate (L-195, lauric acid: 99% by mass, HLB: 1, manufactured by Mitsubishi Chemical Foods Co., Ltd.) in a concentration of 62.5 mg/ml so that the mass ratio of octreotide acetate to the surfactant was 1/50, and then the octreotide acetate-surfactant composite material was prepared from the resultant mixture in the same manner as in Example 6.

To this composite material was added 1 ml of isopropyl myristate (manufactured by Tokyo Kasei Kogyo Co., Ltd.) so as to disperse the composite material therein, to thereby obtain an S/O type preparation. The S/O type preparation was mixed withDURO-TAK 87-4098 (acrylic copolymer, solid content: 38.5% by mass, manufactured by Henkel AG & Co. KGaA) as an adhesive component in a mass ratio of 1:1. The resultant mixture was mixed thoroughly with a vortex mixer (VORTEX-GENIE 2 Mixer, manufactured by M&S Instruments Inc.) until a homogeneous mixture was obtained, yielding an adhesive solution containing the S/O type preparation. The adhesive solution was applied to a liner layer (liner OPP 40 µm), and was dried at 60°C for about 30 minutes to form an adhesive layer on the liner layer. Subsequently, a support material (sheet of acrylic resin having a thickness of 50 µm) was attached to the surface of the adhesive layer opposite to the surface in contact with the liner layer, to thereby obtain an S/O type skin patch.

### [Test Example 9]

### (Pharmacokinetic study of octreotide acetate in S/O type skin patch)

Six-week-old mice (BALB/c, female) were acclimatized in an environment with free access to water and food for one week. Thereafter the backs of the mice were completely depilated by using a clipper and a depilatory cream three days before the study started. The mice were divided into the following three groups (3 mice per group) to which 0.6 mg of octreotide acetate was administered per mouse.

For S/O type skin patch transdermal administration group, the S/O type skin patch prepared in Example 8 (20 mm in length × 30 mm in width) was attached to the back of each mouse and secured with a plastic tape, followed by removal after 24 hours. For PBS solution injection group, 100 µl of a PBS solution of octreotide acetate (6 mg/ml) was subcutaneously injected to each mouse. For sustained release preparation injection group, 0.6 mg of Sandostatin LAR^{®} (manufactured by Novartis Pharma AG) was intramuscularly injected into the femoral region of the right hindlimb of each mouse.

Blood samples were collected via the ocular fundus of each mouse at 0.25, 0.5, 1, 2, 6, 12, 24, 48, and 96 hours after the administration. Plasma concentration of octreotide acetate was quantified by LC-MS (manufactured by Shimadzu Corporation). In addition, blood glucose levels before administration, 1 hour after administration, and 24 hours after administration were measured using One Touch Verio View (LifeScan Japan).

### (Results)

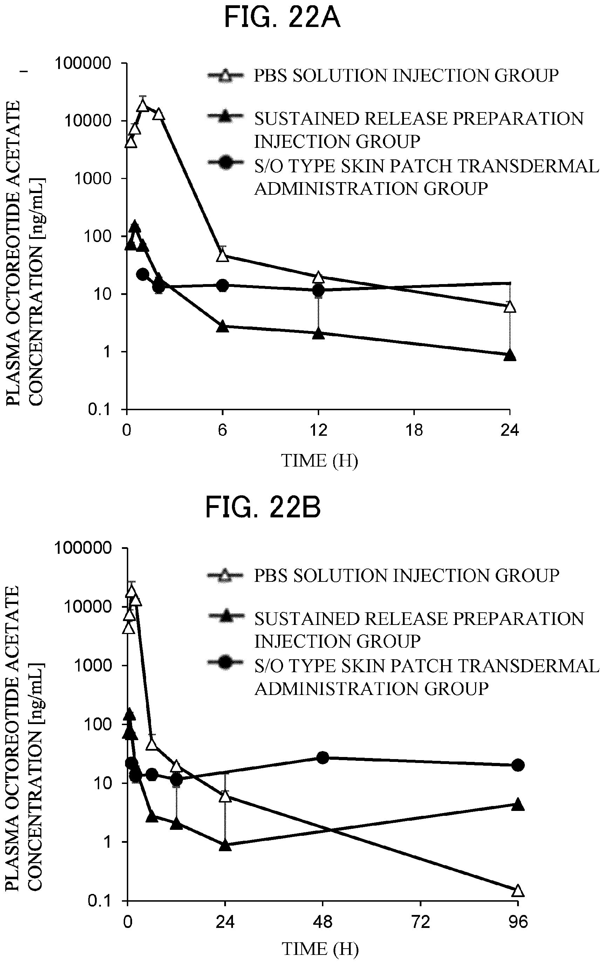

FIG. 22A and FIG. 22B show the time courses of the plasma concentration of octreotide acetate from 0 to 24 hours and from 0 to 96 hours, respectively. At 6 hours after the administration, the plasma concentrations of octreotide acetate in mouse to which the S/O type skin patch was transdermally administered exceeded that of the sustained release preparation injection group, and thereafter stayed at higher plasma concentration level than that of the sustained release preparation injection group. The results of pharmacokinetic analysis are shown in Table 4.

**[Table 4]**

| | Cmax [ng/ml] | Tₘₐₓ [h] | AUC (0-96 h) [ng·h/ml] | AUC (6-96 h) [ng·h/ml] |
|---|---|---|---|---|
| S/O type skin patch transdermal administration group | 27.3 | 24 | 2105.5 | 2022.0 |
| PBS solution injection group | 18544.2 | 1 | 51379.7 | 434.2 |
| Sustained release preparation injection group | 151.8 | 0.5 | 438.2 | 300.8 |

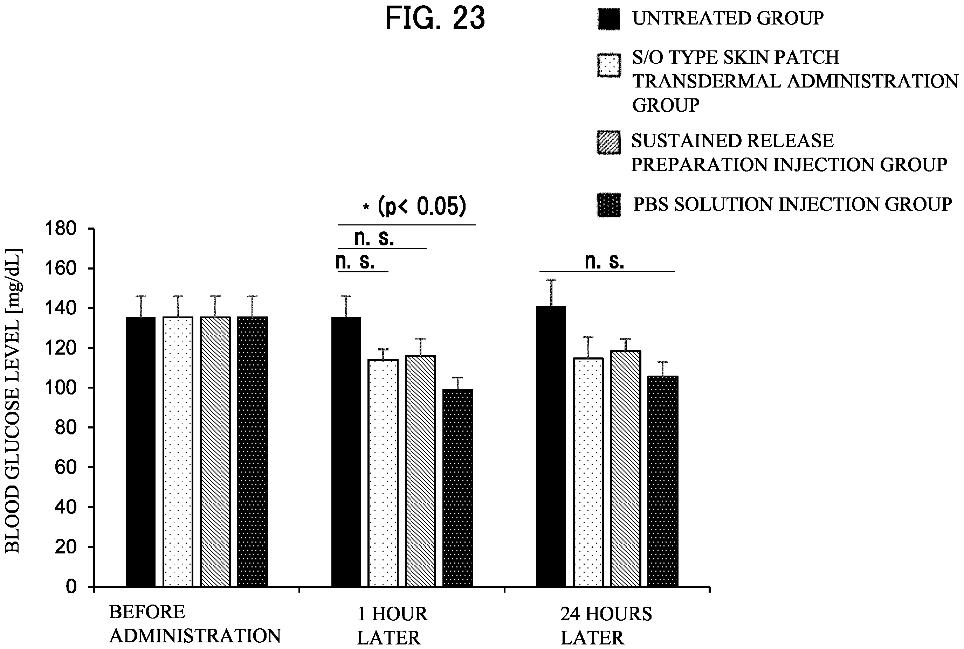

Furthermore, as shown in FIG. 23, a significant decrease in blood glucose level was observed in the PBS solution injection group 1 hour after the administration.

In contrast, it was observed that the time course of the blood glucose level of the S/O type skin patch transdermal administration group was similar to that of the commercially available sustained release preparation injection group, indicating that the side effects were mitigated owing to the sustained release properties.

### [Example 9]

### (Preparation of S/O type skin patch)

One mg of FITC-labeled Tyrosinase-related protein epitope peptide (FITC-KKKGSVYDFFVWL (sequence number 1); hereinafter referred to as "FITC-K-TRP-2") was dissolved in 2 ml of ethanol (10% by volume) to prepare an FITC-K TRP-2 solution. To the FITC-K-TRP-2 solution was added 4 ml of a cyclohexane solution containing sucrose laurate (L-195, lauric acid: 99% by mass, HLB: 1, manufactured by Mitsubishi Chemical Foods Co., Ltd.) in a concentration of 12.5 mg/ml so that the mass ratio of FITC-K-TRP-2 to the surfactant was 1/50, and the resultant mixture was agitated at 26,000 rpm for 2 minutes with a homogenizer. The resultant W/O type emulsion was lyophilized for an entire day, to thereby obtain an FITC-K-TRP-2-surfactant composite material.

To this composite material was added 1 ml of isopropyl myristate (manufactured by Tokyo Kasei Kogyo Co., Ltd.) so as to disperse the composite material therein, to thereby obtain an S/O type preparation. The S/O type preparation was mixed withDURO-TAK 87-4098 (acrylic copolymer, solid content: 38.5% by mass, manufactured by Henkel AG & Co. KGaA) as an adhesive component in a mass ratio of 1:1. The resultant mixture was mixed thoroughly with a vortex mixer (VORTEX-GENIE 2 Mixer, manufactured by M&S Instruments Inc.) until a homogeneous mixture was obtained, yielding an adhesive solution containing the S/O type preparation. The adhesive solution was applied to a liner layer (liner OPP 40 µm), and was dried at 60°C for about 30 minutes to form an adhesive layer on the liner layer. Subsequently, a support material (sheet of acrylic resin having a thickness of 50 µm) was attached to the surface of the adhesive layer opposite to the surface in contact with the liner layer, to thereby obtain an S/O type skin patch (hereinafter sometimes abbreviated as "FITC-K-TRP-2-encapsulated S/O self-adhesive sheet").

### [Test Example 10]

### (Test of FITC-K-TRP-2-encapsulated S/O type self-adhesive sheet for permeability into pig skin)

A test of the FITC-K-TRP-2-encapsulated S/O self-adhesive sheet for permeability into pig skin was performed by using the transdermal absorption testing system shown in FIG. 6. Specifically, a jacketed static Franz diffusion cell for membrane permeability assay (manufactured by CosMED Pharmaceutical) was filled with water, and a piece of pig skin was placed over an opening of the top surface of the diffusion cell. Then, the FITC-K-TRP-2-encapsulated S/O self-adhesive sheet was overlaid on the pig skin so that the adhesive layer was attached to the pig skin, followed by allowing to stand at room temperature for about 24 hours. The surface of the pig skin was rinsed several times with ethanol and PBS. The resulting pig skin was shredded and immersed in 500 µl of extraction solution (PBS: acetonitrile: methanol = 2:1:1 by volume). The resultant mixture was shaken for 24 hours, and the resultant extraction solution was filtered through a 0.2 µm filter. The amount of FITC-K-TRP-2 in the filtrate was quantified by using fluorescence spectrophotometer (LS-55, manufactured by PerkinElmer, Inc.), and the amount of FITC-K-TRP-2 permeated into the pig skin was calculated from the result. As a control, a PBS solution containing 10% ethanol and 21.4 µg of FITC-K-TRP-2, the same amount as that contained in the FITC-K-TRP-2-encapsulated S/O self-adhesive sheet, was applied to another piece of pig skin and the test of permeability was performed.

### (Results)

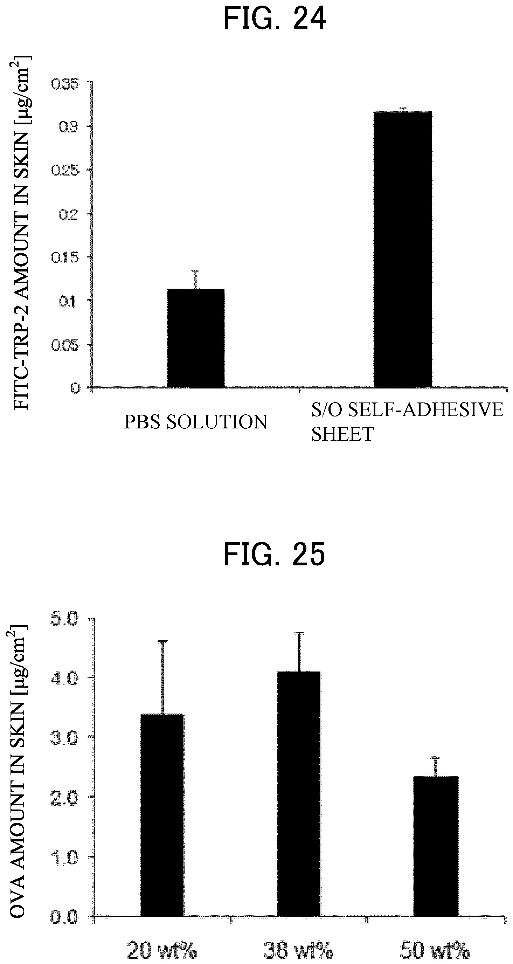

As shown in FIG. 24, it was confirmed that the amount of FITC-K-TRP-2 permeated into the skin from the FITC-K-TRP-2-encapsulated S/O self-adhesive sheet was higher than that of the PBS solution. The permeation rate ([amount of FITC-K-TRP-2 permeated into the skin]/[total amount of FITC-K-TRP-2 originally contained in the self-adhesive sheet] × 100) was found to be 1.9 %.

### [Example 10]

### (Preparation of S/O type skin patch)

To an FITC-OVA solution containing 1 mg of FITC-OVA dissolved in 2 ml of distilled water was added 4 ml of a cyclohexane solution containing sucrose laurate (L-195, lauric acid: 99% by mass, HLB: 1, manufactured by Mitsubishi Chemical Foods Co., Ltd.) in a concentration of 12.5 mg/ml so that the mass ratio of FITC-OVA to the surfactant was 1/50, and the resultant mixture was agitated at 26,000 rpm for 2 minutes with a homogenizer. The resultant W/O type emulsion was lyophilized for an entire day, to thereby obtain a FITC-OVA-surfactant composite material.

To this composite material was added 1 ml of isopropyl myristate (manufactured by Tokyo Kasei Kogyo Co., Ltd.) so as to disperse the composite material therein, to thereby obtain an S/O type preparation. The S/O type preparation was mixed withDURO-TAK 87-4098 (acrylic copolymer, solid content: 38.5% by mass, manufactured by Henkel AG & Co. KGaA) as an adhesive component in a mass ratio of 1:0.41, 1:1, or 1:1.62. The resultant mixture was mixed thoroughly with a vortex mixer (VORTEX- GENIE 2 Mixer, manufactured by M&S Instruments Inc.) until a homogeneous mixture was obtained, yielding an adhesive solution containing the S/O type preparation. The adhesive solution was applied to a liner layer (liner OPP 40 µm), and was dried at 60°C for about 30 minutes to form an adhesive layer on the liner layer. Subsequently, a support material (sheet of acrylic resin having a thickness of 50 µm) was attached to the surface of the adhesive layer opposite to the surface in contact with the liner layer, to thereby obtain an S/O type skin patch (hereinafter sometimes abbreviated as "FITC-OVA-encapsulated S/O self-adhesive sheet") with the content of the adhesive component relative to the total mass of the adhesive component and isopropyl myristate being 20%, 38%, or 50%.

### [Test Example 11]

### (Test of FITC-OVA-encapsulated S/O self-adhesive sheet for permeability into pig skin)

A test of the FITC-OVA-encapsulated S/O self-adhesive sheet for permeability into pig skin was performed by using the transdermal absorption testing system shown in FIG. 6. Specifically, a jacketed static Franz diffusion cell for membrane permeability assay (manufactured by CosMED Pharmaceutical) was filled with water, and a piece of pig skin was placed over an opening of the top surface of the diffusion cell. Then, the FITC-OVA-encapsulated S/O self-adhesive sheet was overlaid on the pig skin so that the adhesive layer was attached to the pig skin, followed by allowing to stand at room temperature for about 24 hours. The surface of the pig skin was rinsed several times with ethanol and PBS. The resulting pig skin was shredded and immersed in 500 µl of extraction solution (PBS: acetonitrile: methanol = 2:1:1 by volume). The resultant mixture was shaken for 24 hours, and the resultant extraction solution was filtered through a 0.2 µm filter. The concentration of FITC-OVA in the filtrate was quantified by using fluorescence spectrophotometer (LS-55, manufactured by PerkinElmer, Inc.), and the amount of the FITC-OVA permeated into the pig skin was calculated from the result.

### (Results)

As shown in FIG. 25, it was observed that the viscosity of the adhesive layer of the FITC-OVA-encapsulated S/O self-adhesive sheet containing 20 % of the adhesive component was low, as a result of which a part of the adhesive layer was left on the liner layer after peeling off the liner layer from the self-adhesive sheet. The FITC-OVA-encapsulated S/O self-adhesive sheet containing 20% of the adhesive component is not suitable for application to the skin because of considerably low adhesion properties. It was also observed that the amount permeated into the pig skin from the FITC-OVA-encapsulated S/O self-adhesive sheet containing 38% of the adhesive component was higher than that of the FITC-OVA-encapsulated S/O self-adhesive sheet containing 50% of the adhesive component.

### [Example 11]

### (Preparation of S/O type skin patch)

To an OVA solution containing 10 mg of OVA dissolved in 4 ml of distilled water was added 8 ml of a cyclohexane solution containing sucrose laurate (L-195, lauric acid: 99% by mass, HLB: 1, manufactured by Mitsubishi Chemical Foods Co., Ltd.) in a concentration of 62.5 mg/ml so that the mass ratio of OVA to the surfactant was 1/50, and the resultant mixture was agitated at 26,000 rpm for 2 minutes with a homogenizer. The resultant W/O type emulsion was lyophilized for an entire day, to thereby obtain an OVA-surfactant composite material.

To this composite material was added 1.67 ml of isopropyl myristate (manufactured by Tokyo Kasei Kogyo) so as to disperse the composite material therein, to thereby obtain an S/O type preparation. The S/O type preparation was mixed with DURO-TAK 87-4098 (acrylic copolymer, solid content: 38.5% by mass, manufactured by Henkel AG & Co. KGaA) as an adhesive component in a mass ratio of 1:1. The resultant mixture was mixed thoroughly with a vortex mixer (VORTEX- GENIE 2 Mixer, manufactured by M&S Instruments Inc.) until a homogeneous mixture was obtained, yielding an adhesive solution containing the S/O type preparation. The adhesive solution was applied to a liner layer (liner OPP 40 µm), and was dried at 60°C for about 30 minutes to form an adhesive layer on the liner layer. Subsequently, a support material (sheet of acrylic resin having a thickness of 50 µm) was attached to the surface of the adhesive layer opposite to the surface in contact with the liner layer, to thereby obtain an S/O type skin patch (hereinafter sometimes abbreviated as "OVA-encapsulated S/O self-adhesive sheet").

Separately, to the OVA-surfactant composite material were added 0.9 ml of isopropyl myristate (manufactured by Tokyo Kasei Kogyo) and 0.1 ml of glyceryl monooleate (MGO) so as to disperse the composite material therein, to thereby obtain an MGO-containing S/O type preparation. Subsequently, this preparation was formed into a self-adhesive sheet with a release liner in the same manner as the OVA-encapsulated S/O self-adhesive sheet, to thereby obtain a skin patch of the MGO-containing S/O type preparation (hereinafter sometimes abbreviated as "OVA-encapsulated MGO self-adhesive sheet").

### [Test Example 12]

### (Animal immunization test using OVA-encapsulated S/O self-adhesive sheet and OVA-encapsulated MGO self-adhesive sheet)

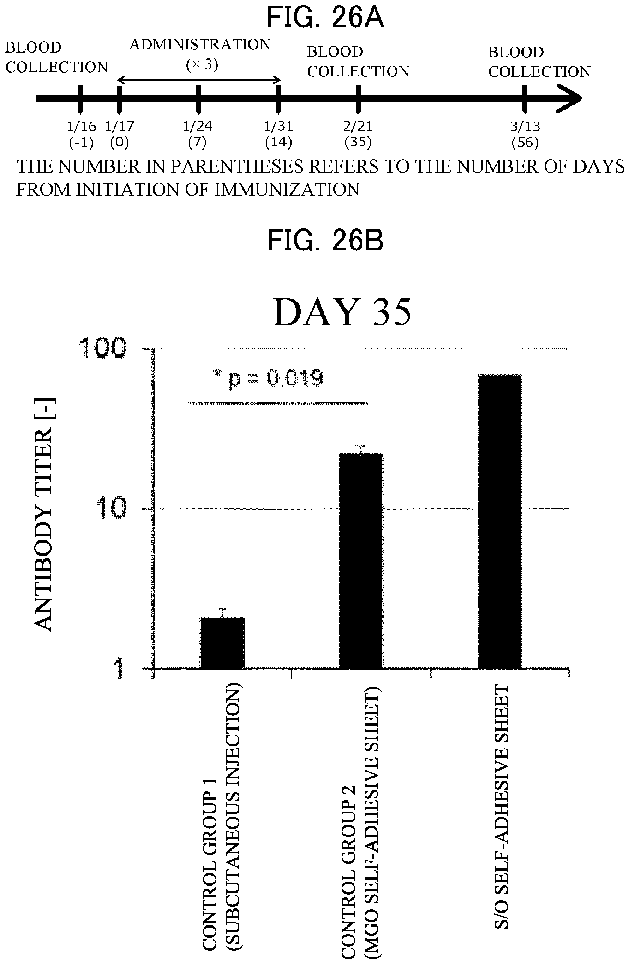

Miniature pig immunization test was performed in accordance with the time schedule shown in FIG. 26A. Specifically, first, hairs at the base of the ear of CLAWN miniature pig was shaved. Subsequently, blood was collected via the anterior aortic sinus and external jugular vein on the day before the immunization test started. Subsequently, on the following day, the OVA-encapsulated S/O self-adhesive sheet obtained in Example 11 was attached to the shaved base of the ear. The OVA-encapsulated S/O self-adhesive sheet was protected by Derm-Smooth Film Roll and Corban Self-Adherent Elastic Bandage Kinesiology Tape (manufactured by Nichiban Corporation) and was further secured with stockinette so as to occlusively patch the self-adhesive sheet for three days. The OVA-encapsulated S/O self-adhesive sheet was replaced with new ones on day 8 and day 15 from the start of the immunization test, administered three times in total. Blood was collected via the anterior aortic sinus and external jugular vein on day 35 and day 56 from the start of the immunization test. As a control 1, a PBS solution containing 3 mg of OVA, the same amount as the S/O self-adhesive sheet, was injected subcutaneously to the miniature pig. Administrations were performed at the same time points as the time schedule for administration of the OVA-encapsulated S/O self-adhesive sheet, administered three times in total. Additionally, as a control 2, OVA-encapsulated MGO self-adhesive sheet containing 3 mg of OVA, the same amount as that contained in the OVA-encapsulated S/O self-adhesive sheet, was attached to the shaved back of mouse in a similar manner to the OVA-encapsulated S/O self-adhesive sheet. Administrations were performed at the same time points as the time schedule for administration of the OVA-encapsulated S/O self-adhesive sheet, administered three times in total. The amount of anti-OVA antibody in the blood was quantified by ELISA in accordance with the following procedure.

First, to a 96-well microplate (maxisorp nunc-immuno plate, manufactured by Thermo Fisher Scientific, Inc.) was added an aqueous solution containing 5.0 mg/ml of OVA in Milli-Q water at 100 µl/well, and the plate was incubated at 4°C overnight. Subsequently, in order to avoid non-specific adsorption to the plate, a PBS solution containing 2% by mass of BSA was added to each well at 200 µl/well for blocking, followed by incubation at 37°C for 2 hours. A serum diluted with a PBS solution containing 2% by mass of BSA at an appropriate dilution ratio was added to the plate at 20 µl/well, followed by incubation at 37°C for 2 hours. Subsequently, a solution of horseradish peroxidase (HRP)-conjugated secondary antibody (Anti-swine IgG-HRP, manufactured by Rockland Immunochemicals, Inc.) (1 mg/ml) was 20,000-fold diluted with a PBS solution containing 2% by mass of BSA, and the resultant solution was added to the plate at 100 µl/well, followed by incubation at 37°C for 2 hours. Subsequently, 3,3',5,5'-tetramethylbenzidine (TMB, manufactured by Thermo Fisher Scientific, Inc.) was added to each well at 100 µl/well, followed by incubation at 37°C for 30 min. Subsequently, 1M HCl was added to each well at 50 µl/well. The O.D. value at 450 nm was read, and the antibody titer was calculated therefrom.

### (Results)

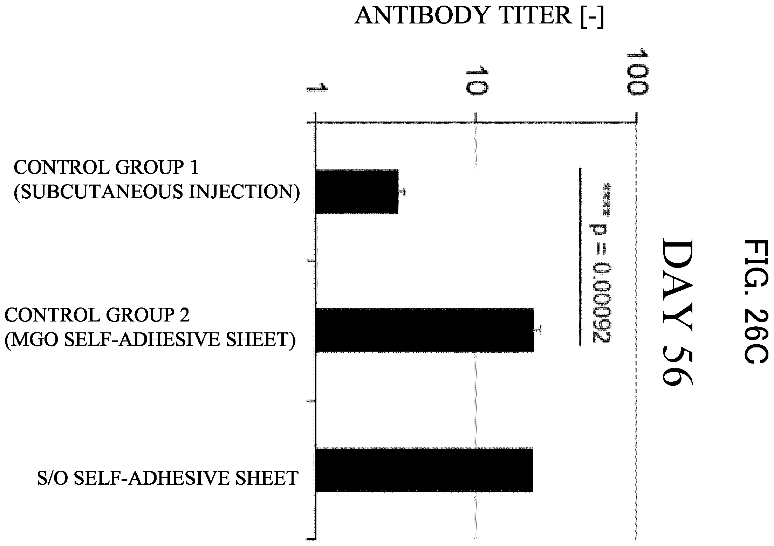

FIG. 26B and FIG. 26C show the antibody titers at day 35 and day 56, respectively. It was clearly shown that, on day 56, the amount of anti-OVA antibody induced by transdermal administration of the OVA-encapsulated S/O self-adhesive sheet was significantly higher than that of the control group 1 to which the PBS solution containing 3 mg of OVA, the same amount as that contained in the OVA-encapsulated S/O self-adhesive sheet, was subcutaneously injected, and was equivalent to that of the control group 2 to which the OVA-encapsulated MGO self-adhesive sheet was transdermally administered.

### [Example 12]

### (Preparation of MGO-containing S/O type skin patch)

To an FITC-OVA solution containing 10 mg of FITC-OVA dissolved in 4 ml of distilled water was added 8 ml of a cyclohexane solution containing sucrose laurate (L-195, lauric acid: 99% by mass, HLB: 1, manufactured by Mitsubishi Chemical Foods Co., Ltd.) in a concentration of 62.5 mg/ml so that the mass ratio of FITC-OVA to the surfactant was 1/50, and the resultant mixture was agitated at 26,000 rpm for 2 minutes with a homogenizer. The resultant W/O type emulsion was lyophilized for an entire day, to thereby obtain a FITC-OVA-surfactant composite material.

MGO was dissolved in isopropyl myristate so that the MGO concentration was 0, 1, 10, or 25% by mass. One ml of the resultant solution was added to the FITC-OVA-surfactant composite material so as to disperse the composite material therein, to thereby obtain MGO-containing S/O type preparations with different contents of MGO.

The MGO-containing S/O type preparation was mixed with DURO-TAK 87-4098 (acrylic copolymer, solid content: 38.5% by mass, manufactured by Henkel AG & Co. KGaA) as an adhesive component in a mass ratio of 1:1. The resultant mixture was mixed thoroughly with a vortex mixer (VORTEX- GENIE 2 Mixer, manufactured by M&S Instruments Inc.) until a homogeneous mixture was obtained, yielding an adhesive solution containing the S/O type preparation. The adhesive solution was applied to a liner layer (liner OPP 40 µm), and was dried at 60°C for about 30 minutes to form an adhesive layer on the liner layer. Subsequently, a support material (sheet of acrylic resin having a thickness of 50 µm) was attached to the surface of the adhesive layer opposite to the surface in contact with the liner layer, to thereby obtain FITC-OVA-encapsulated MGO self-adhesive sheets with different contents of MGO.

### [Test Example 13]

(Test of FITC-OVA-encapsulated MGO self-adhesive sheet for permeability into pig skin)

A test of the FITC-OVA-encapsulated MGO self-adhesive sheets with different contents of MGO for permeability into pig skin was performed by using the transdermal absorption testing system shown in FIG. 6. Specifically, a jacketed static Franz diffusion cell for membrane permeability assay (manufactured by CosMED Pharmaceutical) was filled with water, and a piece of pig skin was placed over an opening of the top surface of the diffusion cell. Then, the FITC-OVA-encapsulated MGO self-adhesive sheet was overlaid on the pig skin so that the adhesive layer was attached to the pig skin, followed by allowing to stand at room temperature for about 24 hours. The surface of the pig skin was rinsed several times with ethanol and PBS. The resulting pig skin was shredded and immersed in 500 µl of extraction solution (PBS: acetonitrile: methanol = 2:1:1 by volume). The resultant mixture was shaken for 24 hours, and the resultant extraction solution was filtered through a 0.2 µm filter. The concentration of FITC-OVA in the filtrate was quantified by using fluorescence spectrophotometer (LS-55, manufactured by PerkinElmer, Inc.), and the amount of the FITC-OVA permeated into the pig skin was calculated from the result.

### (Results)

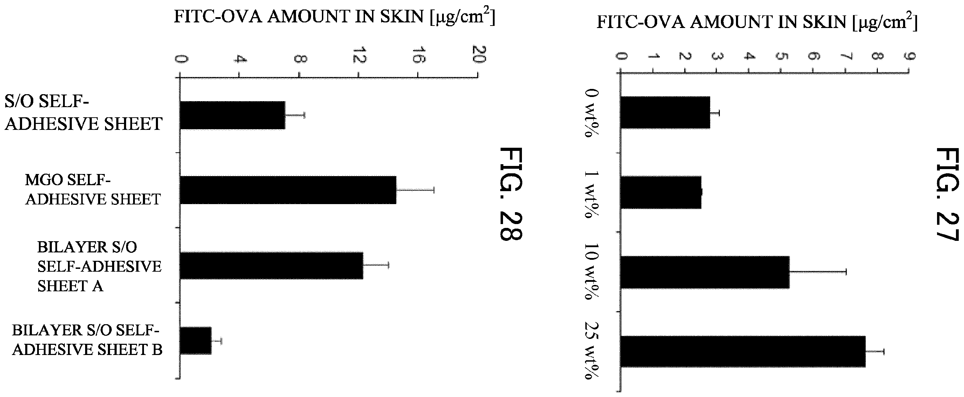

As shown in FIG. 27, the amount of FITC-OVA permeated into the pig skin was increased through the addition of skin permeation promoter, namely, MGO. It was also confirmed that the permeated amount of FITC-OVA was increased as the MGO content increased. Note that in the case of the FITC-OVA-encapsulated MGO self-adhesive sheet containing 25% by mass of MGO, a small part of the adhesive layer was left on the liner layer after the liner layer was peeled off from the adhesive layer. These results indicate that the MGO content of 10 to 20% by mass is feasible.

### [Example 13]

### (Preparation of bilayer S/O type skin patch)

To an FITC-OVA solution containing 10 mg of FITC-OVA dissolved in 4 ml of distilled water was added 8 ml of a cyclohexane solution containing sucrose laurate (L-195, lauric acid: 99% by mass, HLB: 1, manufactured by Mitsubishi Chemical Foods Co., Ltd.) in a concentration of 62.5 mg/ml so that the mass ratio of FITC-OVA to the surfactant was 1/50, and the resultant mixture was agitated at 26,000 rpm for 2 minutes with a homogenizer. The resultant W/O type emulsion was lyophilized for an entire day, to thereby obtain a FITC-OVA-surfactant composite material. To this composite material was added 1 ml of isopropyl myristate (manufactured by Tokyo Kasei Kogyo Co., Ltd.) so as to disperse the composite material therein, to thereby obtain an S/O type preparation. The S/O type preparation was mixed with DURO-TAK 87-4098 as an adhesive component in a mass ratio of 1:1. The resultant mixture was mixed thoroughly with a vortex mixer (VORTEX- GENIE 2 Mixer, manufactured by M&S Instruments Inc.) until a homogeneous mixture was obtained, yielding an adhesive solution containing the S/O type preparation.

Subsequently, a bilayer FITC-OVA-encapsulated S/O self-adhesive sheet including an adhesive layer and a promoter layer was prepared in accordance with the following procedure. For the adhesive layer, an adhesive solution containing the S/O type preparation was used. For the promoter layer, a mixture of a solution containing 10% by mass of MGO in isopropyl myristate with DURO-TAK 87-4098 in a mass ratio of 1:1 (hereinafter sometimes abbreviated as "promoter solution") was used.

### (Preparation of FITC-OVA-encapsulated bilayer S/O self-adhesive sheet A)

The adhesive solution was applied to a liner layer (liner OPP 40 µm), and was dried at 60°C for about 30 minutes to form an adhesive layer on the liner layer. Subsequently, the promoter solution was applied thereon and dried at 60°C for about 30 minutes to form a promoter layer on the adhesive layer. A support material (sheet of acrylic resin having a thickness of 50 µm) was attached to the surface of the promoter layer facing away from the liner layer, to thereby obtain an FITC-OVA-encapsulated bilayer S/O self-adhesive sheet A.

### (Preparation of FITC-OVA-encapsulated bilayer S/O self-adhesive sheet B)

The promoter solution was applied onto a liner layer (liner OPP 40 µm), and was dried at 60°C for about 30 minutes to form a promoter layer on the liner layer. Subsequently, the adhesive solution was applied thereon and dried at 60°C for about 30 minutes to form an adhesive layer on the promoter layer. A support material (sheet of acrylic resin having a thickness of 50 µm) was attached to the surface of the adhesive layer facing away from the liner layer, to thereby obtain an FITC-OVA bilayer encapsulated S/O self-adhesive sheet B.

### [Test Example 14]

### (Test of bilayer S/O skin patch for permeability into pig skin)

A test of FITC-OVA-encapsulated bilayer S/O self-adhesive sheet for permeability into pig skin was performed by using the transdermal absorption testing system shown in FIG. 6. Specifically, a jacketed static Franz diffusion cell for membrane permeability assay (manufactured by CosMED Pharmaceutical) was filled with water, and a piece of pig skin was placed over an opening of the top surface of the diffusion cell. Then, the FITC-OVA bilayer encapsulated S/O self-adhesive sheet was overlaid on the pig skin so that the surface of the layer which had been in contact with the liner layer was attached to the pig skin, followed by allowing to stand at room temperature for about 24 hours. The surface of the pig skin was rinsed several times with ethanol and PBS. The resulting pig skin was shredded and immersed in 500 µl of extraction solution (PBS: acetonitrile: methanol = 2:1:1 by volume). The resultant mixture was shaken for 24 hours, and the resultant extraction solution was filtered through a 0.2 µm filter. The concentration of FITC-OVA in the filtrate was quantified by using fluorescence spectrophotometer (LS-55, manufactured by PerkinElmer, Inc.), and the amount of the FITC-OVA permeated into the pig skin was calculated from the result. As a control, an FITC-OVA-encapsulated S/O self-adhesive sheet or an FITC-OVA-encapsulated MGO self-adhesive sheet containing 10% by mass of MGO relative to isopropyl myristate was placed on pig skin and the test of permeability was performed.

### (Results)

As shown in FIG. 28, it was observed that the amount permeated into the pig skin from the FITC-OVA-encapsulated bilayer S/O self-adhesive sheet A having the adhesive layer on the skin side was higher than that of the FITC-OVA-encapsulated S/O self-adhesive sheet. On the other hand, it was also observed that the amount permeated from the FITC-OVA-encapsulated bilayer S/O self-adhesive sheet B having the promoter layer on the skin side was decreased.

### [Test Example 15]

### (Animal immunization test using bilayer S/O type skin patch)

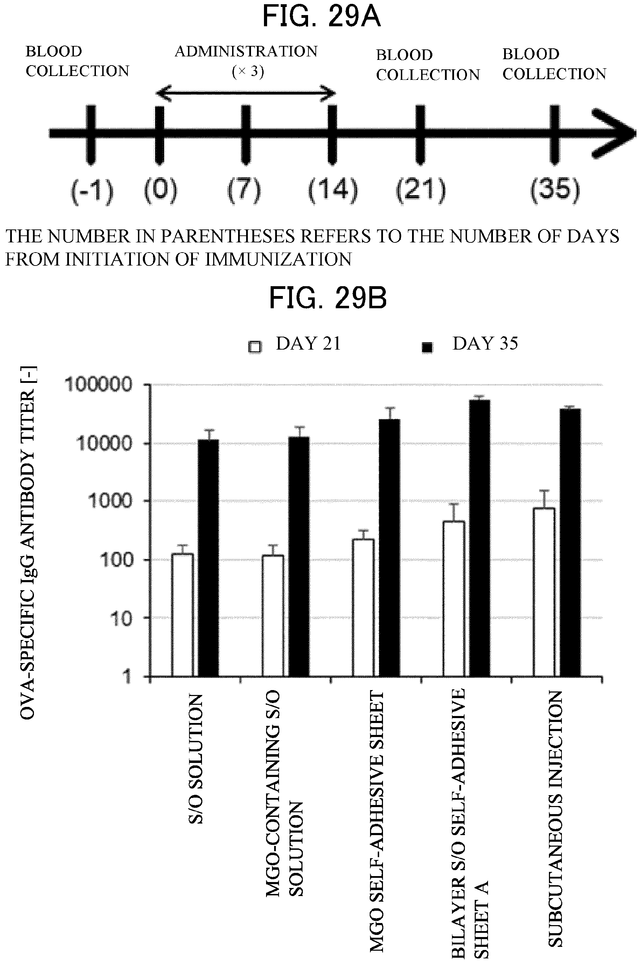

An OVA-encapsulated bilayer S/O self-adhesive sheet A was prepared by repeating the procedure for preparing the FITC-OVA-encapsulated bilayer S/O self-adhesive sheet A described in Example 13 except that OVA (without FITC modification) was used instead of the FITC-OVA. An animal immunization test was performed in accordance with the time schedule shown in FIG. 29A on the self-adhesive sheet A obtained as described above. Specifically, first, hair on the back of mouse was shaved with a clipper three days before the immunization test started. Subsequently, the blood was collected via the caudal vein on the day before the immunization test started. Subsequently, on the following day, the bilayer OVA-encapsulated S/O self-adhesive sheet A was attached to the shaved back skin and secured with kinesiology tape (manufactured by Nichiban Corporation). The bilayer OVA-encapsulated S/O self-adhesive sheet was replaced with new ones on day 7 and day 14 from the start of the immunization test, administered three times in total. On day 21 and day 35 from the start of the immunization test, blood was collected via the caudal vein of the mouse.

As a control group 1, an OVA-encapsulated S/O type preparation (S/O solution) was administered transdermally to the mouse. As a control group 2, an OVA-encapsulated S/O type preparation containing 10% by mass of MGO relative to isopropyl myristate (S/O solution containing MGO) was administered transdermally to the mouse. As a control group 3, an FITC-OVA-encapsulated MGO self-adhesive sheet was administered transdermally to the mouse. As a control 4, a PBS solution containing OVA was injected subcutaneously to the mouse. Each of the control groups contained 30 µg of OVA, the same amount as that contained in the bilayer OVA-encapsulated S/O self-adhesive sheet A. For each of the control groups the administration was performed three times in total in accordance with the same time schedule. The amount of anti-OVA antibody in the blood was examined by ELISA as in the Test example 3.

### (Results)

As shown in FIG. 29B, on day 35, the OVA-encapsulated bilayer S/O self-adhesive sheet A exhibited the highest OVA-specific IgG antibody titer, meaning that the OVA-specific IgG antibody titer induced by the OVA-encapsulated bilayer S/O self-adhesive sheet A exceeded that of the group received subcutaneous injection of PBS solution containing OVA.

The embodiments described above are only for illustrating the present disclosure and are not meant to be limiting the scope of the present disclosure. In other words, the scope of the present disclosure is not defined by the embodiments but is defined by the claims. In addition, various modifications made within the scope defined by the claims and within the scope equivalent thereto that falls within the meaning of the present disclosure are considered as being within the scope of the present disclosure.

This application claims the benefit of Japanese Patent Application No. 2019-189199 filed on October 16, 2019, Japanese Patent Application No. 2020-056321 filed on March 26, 2020, and Japanese Patent Application No. 2020-140563 filed on August 24, 2020, of which the entirety of the disclosures is incorporated by reference herein.

### Industrial Applicability

The transdermal absorption-type patch according to the present embodiment is capable of allowing proteins having molecular weights greater than 1,000 to penetrate through the skin to be absorbed into the body.

### Reference Signs List

- 1: Support material
- 2: Adhesive layer
- 2a, 5: Composite material
- 3: Liner layer
- 4: Promotor layer
- 5a: Leuprorelin
- 5b: Surfactant
- 6: Oil phase
- 10, 20, 30, 40: Transdermal absorption-type patch
- 50: Transdermal absorption agent

## Claims

1. A transdermal absorption-type patch, comprising:
a support material; and
an adhesive layer laminated on the support material, wherein
the adhesive layer comprises:
a solid composite material, the solid composite material being an active ingredient with a molecular weight of 800 or greater enclosed by a surfactant;
an oil phase; and
an adhesive agent containing an acrylic elastomer,
a content of the acrylic elastomer is 30% to 70% by mass based on a total mass of the acrylic elastomer and the oil phase, and
the composite material forms a solid-in-oil type particle dispersed in the oil phase.

2. The transdermal absorption-type patch according to claim 1, wherein the content of the acrylic elastomer is 40% to 50% by mass based on the total mass of the acrylic elastomer and the oil phase.

3. The transdermal absorption-type patch according to claim 1 or 2, wherein the support material contains an acrylic resin.

4. The transdermal absorption-type patch according to any one of claims 1 to 3, further comprising:
a liner layer on the adhesive layer, the liner layer covering the adhesive layer.

5. The transdermal absorption-type patch according to any one of claims 1 to 4, wherein the active ingredient is a protein having a molecular weight of greater than 10,000.

6. The transdermal absorption-type patch according to any one of claims 1 to 5, wherein the active ingredient is a protein having a molecular weight of 30,000 or greater.

7. The transdermal absorption-type patch according to any one of claims 1 to 6, wherein the active ingredient is a bioactive peptide, an antibody, or an antigen.

8. The transdermal absorption-type patch according to any one of claims 1 to 7, further comprising:
a promoter layer disposed between the support material and the adhesive layer, the promoter layer containing a transdermal absorption promoter,
wherein the active ingredient is an antigen.

9. The transdermal absorption-type patch according to anyone of claims 1 to 8, wherein the active ingredient is an antigen, and
the transdermal absorption-type patch is used as a vaccine.

10. The transdermal absorption-type patch according to anyone of claims 1 to 4, wherein the active ingredient is leuprorelin, octreotide, or a salt thereof.
